# EUROPÄISCHE PATENTSCHRIFT

(11) **EP 0 758 644 B1**
(45) Veröffentlichungstag und Bekanntmachung des Hinweises auf die Patenterteilung: **15.10.2003**
(21) Anmeldenummer: 96112498.9
(22) Anmeldetag: 02.08.1996
(51) Int. Cl.: C07C 317/32, A61K 31/18

(54) **Sulfonyl- oder Sulfinyl-benzoyl-guanidin-Derivate**
Sulfonyl- or sulfinyl-benzoyl-guanidine derivatives
Dérivés de sulfonyl- ou sulfinyl-benzoyl-guanidine

(30) Priorität: 11.08.1995 DE 19529612
(43) Veröffentlichungstag der Anmeldung: 19.02.1997
(73) Patentinhaber: MERCK PATENT GmbH, 64293 Darmstadt (DE)
(72) Erfinder: Gericke, Rolf, Dr., 64342 Darmstadt (DE); Baumgarth, Manfred, Dr., 64297 Darmstadt (DE); Minck, Klaus-Otto, Dr., 64372 Ober-Ramstadt (DE); Beier, Norbert, Dr., 64354 Reinheim (DE)

(56) Entgegenhaltungen:
- EP-A- 0 416 499
- EP-A- 0 589 336
- EP-A- 0 604 852
- EP-A- 0 699 663

## Beschreibung

Die Erfindung betrifft ortho-substituierte 4-Sulfonyl- oder 4-Sulfinyl-benzoylguanidin-Derivate der Formel l 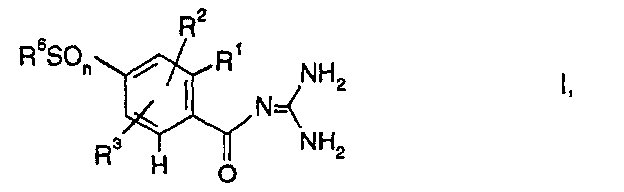 worin
- R¹: A, CF₃, CH₂F, CHF₂, C₂F₅, CN, NO₂ oder Hal,
- R²: -SOₙ-R⁶, -SO₂NR⁴R⁵, NO₂ oder CF₃,
- R³: H, Hal, A, OH, OA, CN, NO₂, CF₃, CH₂F, CHF₂, C₂F₅ oder CH₂CF₃,
- R⁴ und R⁵: jeweils unabhängig voneinander H, A, Cycloalkyl mit 5 bis 7 C-Atomen, Cycloalkylmethyl mit 4 bis 8 C-Atomen, Ph oder -CH₂-Ph,
oder aber
- R⁴ und R⁵: zusammen auch Alkylen mit 4 bis 5 C-Atomen, wobei eine CH₂-Gruppe auch durch CO, O, S, NH, N-A oder N-CH₂-Ph ersetzt sein kann,
- R⁶: A, Ph, Het oder C₃-C₇-Cycloalkyl,
- Het: einen ein- oder zweikernigen gesättigten, ungesättigten oder aromatischen Heterocyclus mit 1 bis 4 N-, O-, und/oder S-Atomen, über N oder C gebunden, der unsubstituiert oder ein-, zwei- oder dreifach durch Hal, SH, SA, NH₂, NHA, NA₂, CF₃, A, OH, OA, CN, NO₂, NHA, NA₂ und/oder Carbonylsauerstoff substituiert sein kann,
- A: Alkyl mit 1 bis 6 C-Atomen,
- Hal: F, Cl, Br oder l,
- Ph: unsubstituiertes oder ein-, zwei- oder dreifach durch A, OA, NR⁴R⁵, F, Cl, Br, I oder CF₃ substituiertes Phenyl und
- n: 1 oder 2,
bedeuten,
sowie deren physiologisch unbedenkliche Salze.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre physiologisch unbedenklichen Salze bei guter Verträglichkeit wertvolle pharmakologische Eigenschaften besitzen.

Bei den neuen Verbindungen handelt es sich um Inhibitoren des zellulären Na⁺/H⁺-Antiporters, d.h. um Wirkstoffe, die den Na⁺/H⁺-Austauschmechanismus der Zellen hemmen (Düsing et al., Med. Klin. 87, 378-384 (1992)) und die somit gute Antiarrhythmika darstellen, die sich insbesondere zur Behandlung von Arrhythmien eignen, die als Folge von Sauerstoffmangel auftreten.

Der bekannteste Wirkstoff der Gruppe der Acylguanidine ist Amilorid. Diese Substanz zeigt jedoch in erster Linie eine blutdrucksenkende und saluretische Wirkung, was insbesondere bei der Behandlung von Herz-Rhythmus-Störungen unerwünscht ist, während die antiarrhythmischen Eigenschaften nur sehr schwach ausgeprägt sind.

Darüber hinaus kennt man strukturell ähnliche Verbindungen beispielsweise aus EP 04 16 499.

EP-A-0 604 852 beschreibt ebenfalls Benzoylguanidine, die sich jedoch durch die Position der Guanidino-Gruppe von den Verbindungen der Formel l unterscheiden.

EP-A-0 589 336 offenbart ähnliche Benzoylguanidine, die im Gegensatz zu den Verbindungen der Formel l keinen ortho-Substituenten Guanidino-Gruppe tragen.

Gegenstand der Erfindung sind Verbindungen der Formel I sowie ihre physiologisch unbedenklichen Salze.

Die erfindungsgemäßen Substanzen der vorliegenden Anmeldung zeigen eine gute kardioprotektive Wirkung und eignen sich daher besonders zur Infarktbehandlung, Infarktprophylaxe und zur Behandlung von Angina pectoris. Ferner wirken die Substanzen allen pathologischen hypoxischen und ischämischen Schädigungen entgegen, so daß die dadurch primär oder sekundär verursachten Krankheiten behandelt werden können. Die Wirkstoffe sind ebenfalls für präventive Anwendungen gut geeignet.

Aufgrund der protektiven Wirkungen dieser Substanzen bei pathologischen hypoxischen oder ischämischen Situationen resultieren daraus weitere Anwendungsmöglichkeiten bei chirurgischen Eingriffen zum Schutz zeitweilig minderversorgter Organe, bei Organtransplantationen zum Schutz der entnommenen Organe, bei angioplastischen Gefäß- oder Herzeingriffen, bei Ischämien des Nervensystems, bei der Therapie von Schockzuständen und zur präventiven Verhinderung der essentiellen Hypertonie.

Ferner können die Verbindungen auch als Therapeutika bei durch Zeltproliferation bedingten Erkrankungen wie Arteriosklerose, diabetische Spätkomplikationen, Tumorerkrankungen, fibrotischen Erkrankungen, insbesondere von Lunge, Leber und Nieren sowie Organhypertrophien und -hyperplasien, eingesetzt werden. Darüber hinaus eignen sich die Substanzen zur diagnostischen Anwendung zur Erkennung von Krankheiten, die von einer gesteigerten Aktivität des Na⁺/H⁺-Antiporters z.B. in Erythrozyten, Thrombozyten oder Leukozyten begleitet werden.

Die Wirkungen der Verbindungen können mit Hilfe an sich bekannter Methoden ermittelt werden, wie sie z.B. von N. Escobales and J. Figueroa in J. Membrane Biol. 120, 41-49 (1991) oder von L. Counillon, W. Scholz, H.J. Lang und J. Pouysségur in Mol. Pharmacol. 44,1041-1045 (1993) angegeben werden.

Als Versuchstiere eignen sich z.B. Mäuse, Ratten, Meerschweinchen, Hunde, Katzen, Affen oder Schweine.

Die Verbindungen können daher als Arzneimittelwirkstoffe in der Humanund Veterinärmedizin verwendet werden. Ferner können sie als Zwischenprodukte zur Herstellung weiterer Arzneimittelwirkstoffe Verwendung finden.

In den angegebenen Formeln bedeutet A eine verzweigte oder unverzweigte Alkylgruppe mit 1-6, bevorzugt 1-4, insbesondere 1, 2 oder 3 C-Atomen, im einzelnen vorzugsweise Methyl, ferner bevorzugt Ethyl, Propyl, Isopropyl, Butyl, Isobutyl, weiterhin bevorzugt sek.-Butyl, tert.-Butyl, Pentyl, Isopentyl (3-Methylbutyl), Hexyl oder Isohexyl (4-Methylpentyl).

R¹ bedeutet vorzugsweise A, insbesondere Methyl oder Ethyl, CF₃ oder Hal, insbesondere F oder Cl. Ferner aber auch vorzugsweise CH₂F, CHF₂ oder C₂F₅. Besonders bevorzugt ist R¹ Methyl oder Ethyl.

R² bedeutet vorzugsweise A-SO₂, NO₂ oder CF₃. Besonders bevorzugt steht R² für H₃C-SO₂-.

R³ bedeutet vorzugsweise H, aber auch A, CF₃, Cl, Br, F, CN, OA und NO₂. Einer der beiden Reste R² und R³ steht vorzugsweise in 3- oder 5-Position der Benzoylgruppe. Sofern einer der Reste A-SO₂- bedeutet, so befindet sich dieser vorzugsweise in der meta-Position zur Benzoylguanidin-Gruppe.

R⁴ bedeutet vorzugsweise, ebenso wie R⁵, H oder A.

Falls R⁴ und R⁵ zusammen Alkylen bedeuten, so ist die Alkylengruppe vorzugsweise unverzweigt, im einzelnen bevorzugt -(CH₂)ₖ-, wobei k 4 oder 5 bedeutet; aber auch bevorzugt -(CH₂)₂-O-(CH₂)₂-, -(CH₂)₂-NH-(CH₂)₂-, -(CH₂)₂-NA-(CH₂)₂-, -CH₂-O-(CH₂)₂-, -CH₂-NH-(CH₂)₂-, oder -CH₂-NA-(CH₂)₂- bzw. -CO-(CH₂)₃-, -CO-(CH₂)₄- oder -CH₂-CO-(CH₂)₂.

Ph bedeutet vorzugsweise unsubstituiertes oder einfach durch Cl, F, A, OA, NH₂, NHA, NA₂ oder CF₃ substituiertes Phenyl.

R⁶ steht bevorzugt für A, oder aber vorzugsweise auch für unsubstituiertes Phenyl, oder einfach durch A, Hal oder CF₃ substituiertes Phenyl, ferner auch bevorzugt für Cycloalkyl mit 5-7 C-Atomen. R⁶ bedeutet bevorzugt C₁ bis C₆-Alkyl oder C₃ bis C₇-Cycloalkyl. Sofern R⁶ nichtcyclisch ist, steht der Rest vorzugsweise für einen der Alkylreste, die auch für A bevorzugt sind. Besonders bevorzugte Cycloalkylreste für die R⁶ stehen kann, sind Cyclopropyl, Cyclopentyl, Cyclohexyl oder deren einfach durch A, insbesondere Methyl, Ethyl oder Isopropyl, substituierten Abkömmlinge.

Die Variable n steht für 1 oder 2.

Hal bedeutet vorzugsweise F, Cl oder Br.

Het ist vorzugsweise 2- oder 3-Furyl, 2- oder 3-Thienyl, 1-, 2- oder 3-Pyrrolyl, 1-, 2-, 4- oder 5-lmidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, *2-,* 4-, 5- oder 6-Pyrimidinyl, weiterhin bevorzugt 1,2,3-Triazol-1-, -4-oder-5-yl, 1,2,4-Triazol-1-, -3-oder-5-yl, 1- oder 5-Tetrazolyl, 1,2,3-Oxadiazol-4- oder-5-yl, 1,2,4-Oxadiazol-3-oder -5-yl, 1,3,4-Thiadiazol-2- oder-5-yl, 1,2,4-Thiadiazol-3- oder -5-yl, 1,2,3-Thiadiazol-4- oder -5-yl, 2-, 3-, 4-, 5- oder 6-2H-Thiopyranyl, 2-, 3-oder 4-4H-Thiopyranyl, 3- oder 4-Pyridazinyl, Pyrazinyl, 2-, 3-, 4-, 5-, 6-oder 7-Benzofuryl, 2-, 3-, 4-, 5-, 6- oder 7-Benzothienyl, 1-, 2-, 3-, 4-, 5-, 6- oder 7-Indolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-,4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolinyl, 1-, 3-, 4-, 5-, 6-, 7- oder 8-Isochinolinyl, 1-, 2-, 3-, 4- oder 9-Carbazolyl, 1-, 2-, 3-, 4-, 5-, 6-, 7-, 8- oder 9-Acridinyl, 3-, 4-, 5-, 6-, 7- oder 8-Cinnolinyl, 2-, 4-, 5-, 6- 7- oder 8-Chinazolinyl. Die heterocyclischen Reste können auch teilweise oder vollständig hydriert sein. Het kann also z.B. auch bedeuten 2,3-Dihydro-2-, -3-, -4- oder-5-furyl, 2,5-Dihydro-2-, -3-, -4- oder-5-furyl, Tetrahydro-2- oder -3-furyl, 1,3-Dioxolan-4-yl, Tetrahydro-2- oder-3-thienyl, 2,3-Dihydro-1-, -2-, -3-, -4-oder -5-pyrrolyl, 2,5-Dihydro-1-, -2-, -3-, -4- oder-5-pyrrolyl, 1-, 2- oder 3-Pyrrolidinyl, Tetrahydro-1-, -2- oder-4-imidazolyl, 2,3-Dihydro-1-,-2-, -3-, -4- oder -5-pyrazolyl, Tetrahydro-1-, -3- oder -4-pyrazolyl, 1,4-Dihydro-1-, -2-, -3-oder-4-pyridyl, 1,2,3,4-Tetrahydro-1-,-2-,-3-, -4-, -5- oder -6-pyridyl, 1,2,3,6-Tetrahydro-1-, -2-, -3-, -4-, -5- oder-6-pyridyl, 1-, 2-, 3-oder 4-Piperidinyl, 2-, 3- oder 4-Morpholinyl, Tetrahydro-2-, -3- oder-4-pyranyl, 1,4-Dioxanyl, 1,3-Dioxan-2-, -4- oder-5-yl, Hexahydro-1-, -3-oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolinyl, 1,2,3,4-Tetrahydro-1-, -2-, -3-, -4-, -5-, -6-, -7- oder 8-isochinolinyl.

Allgemein gilt, daß sämtliche Reste wie z.B. Het oder Ph, die mehrfach auftreten, gleich oder verschieden sein können, d.h. unabhängig voneinander sind.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die nachstehenden Formeln la bis Ih ausgedrückt werden, die der Formel I entsprechen und worin die nicht näher bezeichneten Reste die bei der Formel I angegebene Bedeutung haben, worin jedoch
- in la: R¹ A und R²-SO₂-A oder NO₂ bedeuten;
- in lb: R¹ A und R⁶ verzweigtes oder unverzweigtes Alkyl mit 1 bis 6 C-Atomen bedeuten;
- in lc: R¹ A und R⁶ Cycloalkyl mit 5 bis 7 C-Atomen bedeuten;
- in Id: R⁶ verzweigtes oder unverzweigtes Alkyl mit 1-6 C-Atomen, R²-SO₂-A, CF₃ oder NO₂ und R¹ Methyl oder Ethyl bedeuten;
- in le: R⁶ Methyl, Ethyl, Propyl, Isopropyl, Butyl, Cycloalkyl mit 3 bis 7 C-Atomen, oder Phenyl, welches unsubstituiert oder einfach durch Cl, Br, Methyl oder CF₃ substituiert ist, bedeutet und R² in meta-Position zur Amidgruppe steht und -SO₂-A bedeuten;
- in If: R¹ und R² in para-Stellung zueinander sind und R¹ A und R² -SO₂-A bedeuten;
- in lg: R⁶ Phenyl oder Methyl, n = 2, R¹ Methyl oder Ethyl und R² in meta-Position zur Amidgruppe steht und -SO₂-A bedeuten;
- in Ih: R¹ Methyl oder Ethyl, R²SO₂-CH₃ und R⁶ Methyl, Ethyl, Propyl, Isopropyl, Phenyl, Cyclopentyl oder Cyclohexyl bedeuten.

Ferner sind alle in la-lh genannten Verbindungen besonders bevorzugt, bei denen gleichzeitig n = 2 ist.

Gegenstand der Erfindung ist ferner ein Verfahren zur Herstellung der Verbindungen der Formel I gemäß Anspruch 1, sowie von deren Salzen, dadurch gekennzeichnet, daß man eine Verbindung der Formel II 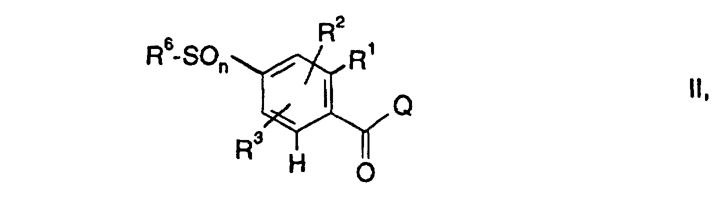 worin R¹, R², R³,R⁶ und n die zuvor angegebenen Bedeutungen haben
und
- Q: Cl, Br, OA, O-CO-A, O-CO-Ph, OH oder eine andere reaktionsfähige veresterte OH-Gruppe bzw. leicht nucleophil substituierbare Abgangsgruppe bedeutet,
mit Guanidin umsetzt,
oder daß man ein Benzoylguanidin der Formel lll 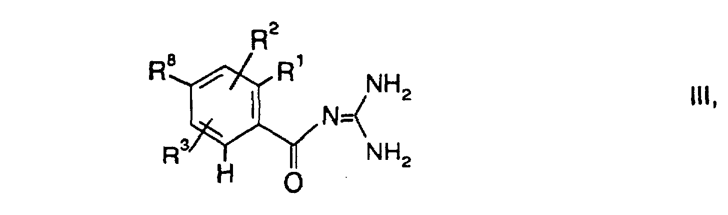 worin R¹, R² und R³ die zuvor angegebenen Bedeutungen besitzen, und
- R⁸: F, Cl, Br, l oder H
bedeutet,
mit einer Verbindung der Formel IV

R⁶-SOₙ-X IV

worin
- R⁶: die angegebene Bedeutung besitzt und
- X: H, Cl, Br oder l bedeutet,
in Gegenwart eines Katalysators, nach vorheriger Metallierung bzw. Transmetallierung, umsetzt,
oder daß man ein Benzoylguanidin der Formel V, 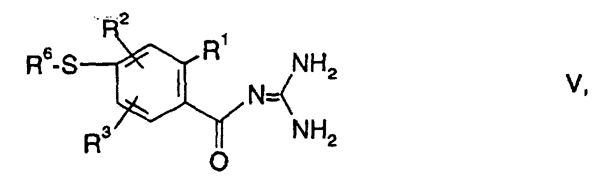 mit einem geeigneten Oxidationsmittel umsetzt,
oder daß man eine sonst der Formel I entsprechende Verbindung, die jedoch anstelle eines oder mehrerer Wasserstoffatome eine oder mehrere reduzierbare Gruppe(n) und/oder eine oder mehrere zusätzliche C-Cund/oder C-N-Bindung(en) enthält, mit einem reduzierenden Mittel behandelt,
oder daß man eine sonst der Formel I entsprechende Verbindung, die jedoch anstelle eines oder mehrerer Wasserstoffatome eine oder mehrere solvolysierbare Gruppe(n) enthält, mit einem solvolysierenden Mittel behandelt
und/oder daß man eine erhaltene Base der Formel I durch Behandeln mit einer Säure in eines ihrer Salze umwandelt.

Die Verbindungen der Formel I werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; Organic Reactions, John Wiley & Sons, Inc., New York; sowie in der oben angegebenen Patentanmeldung) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Vorzugsweise Werden Verbindungen der Formel I hergestellt, indem man ein aktiviertes Carbonsäurederivat der Formel II, wobei Q besonders bevorzugt Cl oder -O-CH₃ ist, mit Guanidin umsetzt. Besonders geeignet sind auch Reaktionsvarianten, bei denen die freie Carbonsäure ll (Q = OH) in an sich bekannter Weise zu dem jeweiligen aktivierten Derivat umgesetzt und dieses dann direkt, ohne Zwischenisolierung, mit Guanidin zur Reaktion gebracht wird. Methoden, bei denen eine Zwischenisolierung entbehrlich ist, sind beispielsweise eine Aktivierung mit Carbonyldiimidazol. Dicyclohexylcarbodiimid oder die Mukayama-Variante (Angew. Chem. 91, 788-812 (1979).

Die Carbonsäuren und Carbonsäurederivate der Formel II sind in der Regel bekannt. Sie werden insbesondere aus den entsprechenden Halogenverbindungen durch nucleophile aromatische Substitution hergestellt. Der Austausch Hal → SR⁶ wird bevorzugt auf der Säurestufe durchgeführt. Anschließend wird mit einem Oxidationsmittel wie H₂O₂, Peressigsäure, Perbenzoesäure oder Natriumperborat umgesetzt.

Die Carbonsäuren der Formel II bzw. deren Derivate werden ferner hergestellt, indem man geeignete Benzoesäurederivate der Formel Vl 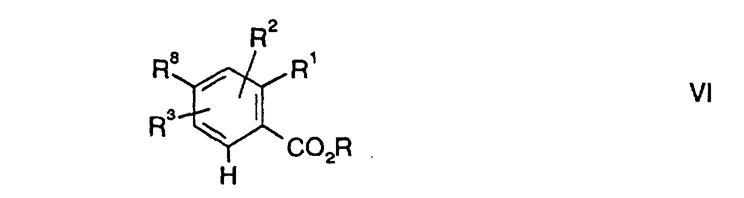 worin R¹, R², R³ und R⁸ die angegebenen Bedeutungen besitzen und R Wasserstoff oder A ist,
metalliert und dann mit eine Alkylsulfonylderivat der Formel IV umsetzt. Eine geeignete Base zur Metallierung ist z.B. Lithiumdiisopropylamid.

Bei den zuvor genannten Kreuzkupplungen setzt man ein Carbonsäureoder ein Esterderivat der Formel VI, worin R⁸ Cl, Br oder I bedeutet, mit einer metallorganischen Alkylverbindung, die in situ durch Metallierung mit einem an sich bekannten Metallierungsreagenz aus einer Verbindung der Formel IV hergestellt wird, in Gegenwart eines geeigneten Metallkatalysators, insbesondere eines der zuvor genannten, um.

Die Umsetzung erfolgt in Analogie zu der Reaktion der Verbindungen lll und IV. Sie wird nachstehend beschrieben.

Die Umsetzung eines reaktionsfähigen Carbonsäurederivates der Formel II mit Guanidin erfolgt in an sich bekannter Weise vorzugsweise in einem protischen oder aprotischen polaren oder unpolaren inerten organischen Lösungsmittel.

Geeignete Lösungsmittel werden nachfolgend für die Umsetzung der Verbindungen III und IV genannt. Besonders bevorzugte Solventien sind jedoch Methanol, THF, Dimethoxyethan, Dioxan oder daraus herstellbare Gemische sowie Wasser. Als Reaktionstemperatur sind beispielsweise Temperaturen zwischen 20° und dem Siedepunkt des Lösungsmittels geeignet. Die Reaktionszeiten liegen zwischen 5 Min. und 12 Std.. Es ist zweckmäßig, bei der Reaktion einen Säurefänger einzusetzen. Hierzu eignen sich jegliche Arten von Basen, die die Reaktion selbst nicht stören. Besonders geeignet ist jedoch die Verwendung von anorganischen Basen wie Kaliumcarbonat oder von organischen Basen wie Triethylamin oder Pyridin oder aber ein Überschuß des Guanidins.

Verbindungen der Formel I gemäß Anspruch 1 können ferner hergestellt werden, indem man ein Benzoylguanidin der Formel III mit einer Verbindung der Formel IV umsetzt. Die Ausgangsstoffe der Formel III können auf einfache Weise durch Umsetzung von entsprechend substituierten Benzoesäuren oder daraus ableitbaren reaktionsfähigen Säurederivaten, wie z.B. Säurehalogeniden, Estern oder Anhydriden, mit Guanidin, unter Reaktionsbedingungen wie sie für die Amidherstellung an sich bekannt und allgemein üblich sind, hergestellt werden. Besonders geeignet sind wiederum solche Reaktionsvarianten, wie sie zuvor für die Umsetzung von Verbindung II mit Guanidin angegeben werden.

Die Verbindungen der Formel IV sind ebenso wie die Methoden zu ihrer Herstellung an sich bekannt. Sofern sie nicht bekannt sind, können sie nach den an sich bekannten Methoden hergestellt werden.

Die Herstellung der Verbindung II sowie die Umsetzung der Verbindung lll mit einer Verbindung der Formel IV erfolgt in an sich bekannter Weise, bevorzugt in einem protischen oder aprotischen polaren inerten organischen Lösungsmittel.

Bei der Herstellung von II oder bei der Umsetzung von III mit IV ist es ebenfalls zweckmäßig, in Gegenwart einer Base oder mit einem Überschuß der basischen Komponente zu arbeiten. Als Basen eignen sich bevorzugt z.B. Alkalimetall- oder Erdalkalimetallhydroxide, -carbonate, -alkoholate oder organische Basen wie Triethylamin oder Pyridin, die auch im Überschuß angewendet werden und dann gleichzeitig als Lösungsmittel dienen können.

Als inerte Lösungsmittel eignen sich insbesondere Alkohole wie Methanol, Ethanol, Isopropanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Nitrile wie Acetonitril; Nitroverbindungen wie Nitromethan oder Nitrobenzol; Ester wie Ethylacetat; Amide wie Phosphorsäurehexamethyltriamid; Sulfoxide wie Dimethylsulfoxid (DMSO); chlorierte Kohlenwasserstoffe wie Dichlormethan, Chloroform, Trichlorethylen, 1,2-Dichlorethan oder Kohlenstofftetrachlorid; Kohlenwasserstoffe wie Benzol, Toluol oder Xylol. Weiterhin eignen sich Gemische dieser Lösungsmittel untereinander.

Eine besonders bevorzugte Arbeitsweise bei der Umsetzung von III mit IV besteht darin, daß man das entsprechende Benzoylguanidin in einem inerten Lösungsmittel, wie z.B. Toluol, suspendiert mit einem Pd-(ll)-Katalysator behandelt und anschließend tropfenweise die gewünschte zuvor transmetallierte Verbindung, z.B. eine Zn-Alkylsulfonylverbindung, der Formel IV zusetzt.

Eine weitere bevorzugte Variante zur Herstellung von Verbindungen der Formel I besteht darin, daß man eine Verbindung der Formel V mit einem Oxidationsmittel zu einer Sulfonylverbindung oxidiert. Geeignete Oxidationsmittel sind z.B. Waserstoffperoxid, Peressigsäure, Perbenzoesäure oder Natriumperborat. Die Verbindungen der Formel V können beispielsweise durch nucleophile aromatische Substitution aus den entsprechenden Halogenverbindungen hergestellt werden, wobei der Halogen/SR⁶-Austausch vorzugsweise auf der Säurestufe erfolgt und anschließend eine Überführung in das Benzoylguanidin vorgenommen wird.

Die Sulfinyl-benzoylguanidine der Formel I (n = 1) liegen in racemischer Form vor und können in Enantiomere gespalten werden. Diese Verbindungen werden sowohl als Racemate, als auch in enantiomerenreiner Form von der Erfindung eingeschlossen.

Weiterhin können die Verbindungen der Formel l erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die sonst der Formel I entsprechen, aber anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, insbesondere solche, die anstelle einer HN-Gruppe eine R'-N-Gruppe tragen, worin R' eine Aminoschutzgruppe bedeutet, und/oder solche, die anstelle des H-Atoms einer Hydroxygruppe eine Hydroxyschutzgruppe trägen, z.B. solche, die der Formel I entsprechen, jedoch anstelle einer OH-Gruppe eine OR"-Gruppe tragen, worin R" eine Hydroxyschutzgruppe bedeutet.

Es können auch mehrere - gleiche oder verschiedene - geschützte Aminound/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an einer anderen Stelle des Moleküls durchgeführt worden ist. Typisch für solche Gruppen sind insbesondere unsubstituierte oder substituierte Acyl-, Aryl- (z.B. 2,4-Dinitrophenyl (DNP)), Aralkoxymethyl- (z.B. Benzyloxymethyl (BOM)) oder Aralkylgruppen (z.B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren im weitesten Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie Phenoxyacetyl; Alkoxycarbonyl wie Methoxycarbonyl, Ethoxycarbonyl, 2,2,2-Trichlorethoxycarbonyl, Isopropoxycarbonyl, tert.-Butoxycarbonyl (BOC), 2-Jodethoxycarbonyl; Aralkyloxycarbonyl wie Benzyloxycarbonyl (CBZ), 4-Methoxybenzyloxycarbonyl, 9-Fluorenylmethoxycarbonyl (FMOC). Bevorzugte Aminoschutzgruppen sind BOC, DNP und BOM, ferner CBZ, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an einer anderen Stelle des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. tert.-Butyl, Benzyl, p-Nitrobenzoyl, p-Toluolsulfonyl und Acetyl, wobei Benzyl und Acetyl besonders bevorzugt sind.

Die als Ausgangsstoffe zu verwendenden funktionellen Derivate der Verbindungen der Formel I können nach üblichen Methoden hergestellt werden, wie sie z.B. in den genannten Standardwerken und Patentanmeldungen beschrieben sind, z.B. durch Umsetzung von Verbindungen, die den Formeln II und III entsprechen, wobei jedoch mindestens eine dieser Verbindungen eine Schutzgruppe anstelle eines H-Atoms enthält.

Das In-Freiheit-Setzen der Verbindungen der Formel I aus ihren funktionellen Derivaten gelingt - je nach der benutzten Schutzgruppe - z.B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich.

Als inerte Lösungsmittel eignen sich vorzugsweise organische, beispielsweise Carbonsäuren wie Essigsäure, Ether wie Tetrahydrofuran (THF) oder Dioxan, Amide wie Dimethylformamid (DMF), halogenierte Kohlenwasserstoffe wie Dichlormethan, ferner auch Alkohole wie Methanol, Ethanol oder Isopropanol sowie Wasser. Ferner kommen Gemische der vorgenannten Lösungsmittel in Frage. Trifluoressigsäüre wird vorzugsweise im Überschuß ohne Zusatz eines weiteren Lösungsmittels verwendet, Perchlorsäure in Form eines Gemisches aus Essigsäure und 70%iger Perchlorsäure im Verhältnis 9:1. Die Reaktionstemperaturen für die Spaltung liegen zweckmäßig zwischen etwa 0 und etwa 50°; vorzugsweise arbeitet man zwischen 15 und 30° (Raumtemperatur).

Die BOC-Gruppe kann z.B. bevorzugt mit 40%iger Trifluoressigsäure in Dichlormethan oder mit etwa 3 bis 5 n HCI in Dioxan bei 15-60° abgespalten weden, die FMOC-Gruppe mit einer etwa 5-20%igen Lösung von Dimethylamin, Diethylamin oder Piperidin in DMF bei 15-50°. Eine Abspaltung der DNP-Gruppe gelingt z.B. auch mit einer etwa 3-10%igen Lösung von 2-Mercaptoethanol in DMF/Wasser bei 15-30°.

Hydrogenolytisch entfernbare Schutzgruppen (z.B. BOM, CBZ oder Benzyl) können z.B. durch Behandeln mit Wasserstoff in Gegenwart eines Katalysators (z.B. eines Edelmetallkatalysators wie Palladium, zweckmäßig auf einem Träger wie Kohle) abgespalten werden. Als Lösungsmittel eignen sich dabei die oben angegebenen, insbesondere z.B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z.B. gut an 5-10%igem Pd-C in Methanol bei 20-30°.

Eine Base der Formel I kann ferner mit einer Säure in das zugehörige Säureadditionssalz übergeführt werden. Für diese Umsetzung kommen Säuren in Frage, die physiologisch. unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalinmono- und disulfonsäuren, Laurylschwefelsäure.

Die Verbindungen der Formel I und ihre physiologisch unbedenklichen Salze könnenzur Herstellung pharmazeutischer Zubereitungen verwendet werden, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel l und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlenhydrate wie Lactose oder Stärke, Magnesiumstearat, Talk, Lanolin, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes, Pasten, Lotionen, Gele, Sprays, Schäume, Aerosole, Lösungen (z.B. Lösungen in Alkoholen wie Ethanol oder Isopropanol, Acetonitril, DMF, Dimethylacetamid, 1,2-Propandiol oder deren Gemischen untereinander und/oder mit Wasser) oder Puder. Die neuen Verbindungen können auch lyophilisiert und die erhaltenen Lyophilisate z.B. zur Herstellung von Injektionspräparaten verwendet werden.

Insbesondere für die topische Anwendung kommen auch liposomale Zubereitungen in Betracht. Die angegebenen Zubereitungen können sterilisiert sein und/oder Hilfsstoffe wie Geleit-, Konservierungs-, Stabilisierungs- und/oder Netzmittel, Emulgatoren, Salze zur Beeinflussung des osmotisches Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I und ihre physiologisch unbedenklichen Salze können an Menschen oder Tiere, insbesondere Säugetiere wie Affen, Hunde, Katzen, Ratten oder Mäuse verabreicht und bei der therapeutischen Behandlung des menschlichen oder tierischen Körpers sowie bei der Bekämpfung von Krankheiten verwendet werden, insbesondere bei der Therapie und/oder Prophylaxe von Störungen des cardiovasculären Systems. Sie eignen sich daher zur Behandlung von Arrhythmien, insbesondere wenn diese durch Sauerstoffmangel hervorgerufen werden, von Angina pectoris, Infarkten, Ischämien des Nervensystems wie z.B. Schlaganfall oder Hirnödeme, von Schockzuständen und zur Präventivbehandlung.

Die Substanzen können ferner als Therapeutika bei Erkrankungen eingesetzt werden, bei denen Zellproliferationen eine Rolle spielen wie Arteriosklerose, diabetische Spätkomplikationen, Tumorerkrankunken, Fibrosen sowie Organhypertrophien und -hyperplasien, insbesondere bei Erkrankungen der Prostata.

Dabei werden die erfindungsgemäßen Substanzen in der Regel in Analogie zu bekannten Antiarrhythmika, z.B. Aprindin, verabreicht, vorzugsweise in Dosierungen zwischen etwa 0,01 und 5 mg, insbesondere zwischen 0,02 und 0,5 mg pro Dosierungseinheit. Die tägliche Dosierung liegt vorzugsweise zwischen etwa 0,0001 und 0,1, insbesondere zwischen 0,0003 und 0,01 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, dem allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

In den nachfolgenden Beispielen bedeutet "übliche Aufarbeitung":

Man gibt, falls erforderlich, Wasser hinzu, säuert an, extrahiert oder wäscht mit einem organischen Lösungsmittel wie Ethylacetat, trennt ab, trocknet die organische Phase über Natriumsulfat, filtriert, dampft ein und reinigt durch Chromatographie und/oder Kristallisation.

### Beispiel 1

900 mg Na werden in 30 ml Methanol gelöst, 3 g Guanidiniumchlorid zugegeben, 30 Minuten bei Raumtemperatur gerührt und filtriert. Nach Entfernen des Lösungsmittels und Waschen mit Toluol wird der Rückstand in 30 ml Ethylenglykolmonomethylether aufgenommen und zu einer Lösung von 1 g 2-Methyl-4,5-di- (methyfsulfonyf)-benzoesäurechlorid [erhältlich durch Umsetzung von 2 -Methyl-4-chlor-5-methylsulfonylbenzoesäure mit Na-methylthiolat, anschließende Oxidation mit Naperborat, Überführung ins Säurechlorid z.B. mit SOCl₂] in 40 ml Ethylenglykolmonomethylether hinzugefügt. Man rührt 2 Stunden bei Raumtemperatur, verdünnt mit 100 ml Eiswasser und gibt 10 ml 1 n HCI Lösung hinzu. Anschließend wird zweimal mit je 50 ml Essigsäureethylester gewaschen und auf einen pH-Wert von 9 eingestellt. Nach üblicher Aufarbeitung und Entfernung des Lösungsmittels erhält man nach Umkristallisieren aus Diethylether das N-Diaminomethylen-2-methyl-4,5-di-(methylsulfonyl)-benzamid, F. 238-240°.

Analog erhält man durch Umsetzung von Guanidin
mit 2-Methyl-4-phenylsulfonyl-5-methylsulfonyl-benzoesäurechlorid das N-Diaminomethylen-2-methyl-4-phenylsulfonyl-5-methylsulfonylbenzamid, F. 235-237°;
mit 2-Methyl-4-ethylsulfonyl-5-methylsulfonyl-benzoesäurechlorid das N-Diaminomethylen-2-methyl-4-ethylsulfonyl-5-methylsulfonylbenzamid;
mit 2-Methyl-4-propylsulfonyl-5-methylsulfonyl-benzoesäurechlorid das N-Diaminomethylen-2-methyl-4-propylsulfonyl-5-methylsulfonylbenzamid;
mit 2-Methyl-4-butylsulfonyl-5-methylsulfonyl-benzoesäurechlorid das N-Diam inomethylen-2-methyl-4-butylsulfonyl-5-methylsulfonylbenzamid;
mit 2-Methyl-4-(2-butylsulfonyl)-5-methylsulfonyl-benzoesäurechlorid das N-Diaminomethylen-2-methyl-4-(2-butylsulfonyl)-5-methylsulfonylbenzamid;
mit 2-Methyl-4-pentylsulfonyl-5-methylsulfonyl-benzoesäurechlorid das N-Diaminomethylen-2-methyl-4-pentylsulfonyl-5-methylsulfonylbenzamid;
mit 2-Methyl-4-(2-pentylsulfonyl)-5-methylsulfonyl-benzoesäurechlorid das N-Diaminomethylen-2-methyl-4-(2-pentylsulfonyl)-5-methylsulfonylbenzamid;
mit 2-Methyl-4-(3-pentylsulfonyl)-5-methylsulfonyl-benzoesäurechlorid das N-Diaminomethylen-2-methyl-4-(3-pentylsulfonyl)-5-methylsulfonylbenzamid;
mit 2-Methyl-4-tert.-butylsulfonyl-5-methylsulfonyl-benzoesäurechlorid das N-Diaminomethylen-2-methyl-4-tert.-butylsulfonyl-5-methylsulfonylbenzamid;
mit 2-Methyl-4-isopropylsulfonyl-5-methylsulfonyl-benzoesäurechlorid das benzamid;
mit 2-Methyl-4-hexylsulfonyt-5-methylsulfonyl-benzoesäurechlorid das N-Diaminomethylen-2-methyl-4-hexylsulfonyl-5-methylsulfonylbenzamid;
mit 2-Methyl-4-cyclopropylsulfonyl-5-methylsulfonyl-benzoesäurechlorid das N-Diaminomethylen-2-methyl-4-cyclopropylsulfonyl-5-methylsulfonylbenzamid;
mit 2-Methyl-4-cyclopentylsulfonyl-5-methylsulfonyl-benzoesäurechlorid das N-Diam inomethylen-2-methyl-4-cyclopentylsulfonyl-5-methylsulfonylbenzamid;
mit 2-Methyl-4-cyclohexylsulfonyl-5-methylsulfonyl-benzoesäurechlorid das N-Diaminomethylen-2-methyl-4-cyclohexylsulfonyl-5-methylsulfonylbenzamid;
mit 2-Methyl-4-(2-methylcyclopentylsulfonyl)-5-methylsulfonyl-benzoesäurechlorid das N-Diaminomethylen-2-methyl-4-(2-methylcyclopentylsulfonyl)-5-methylsulfonyl-benzamid;
mit 2-Methyl-4-cyclobutylsulfonyl-5-methylsulfonyl-benzoesäurechlorid das N-Diaminomethylen-2-methyl-4-cyclobutylsulionyl-5-methylsulfonylbenzamid;
mit 2-Methyl-4-cycloheptylsulfonyl-5-methylsulfonyl-benzoesäurechlorid das N-Diaminomethylen-2-methyl-4-cycloheptylsulfonyl-5-methylsulfonylbenzamid;
mit 2-Ethyl-4-phenylsulfonyl-5-methylsulfonyl-benzoesäurechlorid das N-Diaminomethylen-2-ethyl-4-phenylsulfonyl-5-methylsulfonylbenzamid;
mit 2-Ethyl-4,5-di-(methylsulfonyl)-benzoesäurechlorid das N-Diaminomethylen-2-ethyl-4,5-di-(methylsulfonyl)-benzamid;
mit 2-Ethyl-4-ethylsulfonyl-5-methylsulfonyl-benzoesäurechlorid das N -Diaminomethylen-2-ethyl-4-ethylsulfonyl-5-methylsulfonylbenzamid;
mit 2-Ethyl-4-propylsulfonyl-5-methylsulfonyl-benzoesäurechlorid das N-Diaminomethylen-2-ethyl-4-propylsulfonyl-5-methylsulfonylbenzamid;
mit 2-Ethyl-4-butylsulfonyl-5-methylsulfonyl-benzoesäurechlorid das N-Diaminomethylen-2-ethyl-4-butylsulfonyl-5-methylsulfonylbenzamid;
mit 2-Ethyl-4-(2-butylsulfonyl)-5-methylsulfonyl-benzoesäurechlorid das N-Diaminomethylen-2-ethyl-4-(2-butylsulfonyl)-5-methylsulfonyl-benzamid;
mit 2-Ethyl-4-pentylsulfonyl-5-methylsulfonyl-benzoesäurechlorid das N-Diaminomethylen-2-ethyl-4-pentylsulfonyl-5-methylsulfonylbenzamid;
mit 2-Ethyl-4-(2-penty(sulfonyl)-5-methylsulfonyl-benzoesäurechlorid das N-Diaminomethylen-2-ethyl-4-(2-pentylsulfonyl)-5-methylsulfonylbenzamid;
mit 2-Ethyl-4-(3-pentylsulfonyl)-5-methylsulfonyl-benzoesäurechlorid das N-Diaminomethylen-2-ethyl-4-(3-pentylsulfonyl)-5-methylsulfonylbenzamid;
mit 2-Ethyl-4-tert.-butylsulfonyl-5-methylsulfonyl-benzoesäurechlorid das N-Diaminomethylen-2-ethyl-4-tert.-butylsulfonyl-5-methylsulfonylbenzamid;
mit 2-Ethyl-4-isopropylsulfonyl-5-methylsulfonyl-benzoesäurechlorid das N-Diaminomethylen-2-ethyl-4-isopropylsulfonyl-5-methylsulfonylbenzamid;
mit 2-Ethyl-4-hexylsulfonyl-5-methylsulfonyl-benzoesäurechlorid das N-Diaminomethylen-2-ethyl-4-hexylsulfonyl-5-methylsulfonylbenzamid;
mit 2-Ethyl-4-cyclopropylsulfonyl-5-methylsulfonyl-benzoesäurechlorid das N-Diaminomethylen-2-ethyl-4-cyclopropylsulfonyl-5-methylsulfonylbenzamid;
mit 2-Ethyl-4-cyclopentylsulfonyl-5-methylsulfonyl-benzoesäurechlorid das N-Diaminomethylen-2-ethyl-4-cyclopentylsulfonyl-5-methylsulfonylbenzamid;
mit 2-Ethyl-4-cyclohexylsulfonyl-5-methylsulfonyl-benzoesäurechlorid das N-Diaminomethylen-2-ethyl-4-cyclohexylsulfonyl-5-methylsulfonylbenzamid;
mit 2-Ethyl-4-(2-m ethylcyclopentylsulfonyl)-5-methylsulfonylbenzoesäurechlorid das N-Diaminomethylen-2-ethyl-4-(2-methylcyclopentylsulfonyl)-5-methylsulfonyl-benzamid;
mit 2-Ethyl-4-cyclobutylsulfonyl-5-methylsulfonyl-benzoesäurechlorid das N-Diaminomethylen-2-ethyl-4-cyclobutylsulfonyl-5-methylsulfonylbenzamid;
mit 2-Ethyl-4-cycloheptylsulfonyl-5-methylsulfonyl-benzoesäurechlorid das N-Diaminomethylen-2-ethyl-4-cycloheptylsulfonyt-5-methylsulfonylbenzamid.

### Beispiel 2

Analog Beispiel 1 erhält man durch Umsetzung von 1,8 g 2-Methyl-4-methylsulfonyl-5-nitro-benzoesäurechlorid [erhältlich durch Umsetzung von 2-Methyl-4-brom-5-nitro-benzoesäuremethylester mit Methylthio-zinkchlorid in Gegenwart von Pd-(ll)-[1,1'-bis-(diphenylphosphino)-ferrocen]-chlorid und Cul, anschließende Oxidation mit H₂O₂, Verseifung und Überführung ins Säurechlorid z.B. mit SOCl₂] mit 1,5 g Guanidin in Methanol das N-Diaminomethylen-2-methyl-4-(methylsulfonyl)-5-nitrobenzamid.

Analog erhält man durch Umsetzung von Guanidin
mit 2-Methyl-4-phenylsulfonyl-5-nitro-benzoesäurechlorid das N-Diaminomethylen-2-methyl-4-phenylsulfonyl-5-nitro-benzamid;
mit 2-Methyl-4-ethylsulfonyl-5-nitro-benzoesäurechlorid das N-Diaminomethylen-2-methyl-4-ethylsulfonyl-5-nitro-benzamid;
mit 2-Methyl-4-propylsulfonyl-5-nitro-benzoesäurechlorid das N -Diaminomethylen-2-methyl-4-propylsulfonyl-5-nitro-benzamid;
mit 2-Methyl-4-butylsulfonyl-5-nitro-benzoesäurechlorid das N-Diaminomethylen-2-methyl-4-butylsulfonyl-5-nitro-benzamid;
mit 2-Methyl-4-(2-butylsulfonyl)-5-nitro-benzoesäurechlorid das N-Diaminomethylen-2-methyl-4-(2-butylsulfonyl)-5-nitro-benzamid;
mit 2-Methyl-4-pentylsulfonyl-5-nitro-benzoesäurechlorid das N-Diaminomethylen-2-methyl-4-pentylsulfonyl-5-nitro-benzamid;
mit 2-Methyl-4-(2-pentylsulfonyl)-5-nitro-benzoesäurechlorid das N-Diaminomethylen-2-methyl-4-(2-pentylsulfonyl)-5-nitro-benzamid;
mit 2-Methyl-4-(3-pentylsulfonyl)-5-nitro-benzoesäurechlorid das N-Diaminomethylen-2-methyl-4-(3-pentylsulfonyl)-5-nitro-benzamid;
mit 2-Methyl-4-tert.-butylsulfonyl-5-nitro-benzoesäurechlorid das N-Diaminomethylen-2-methyl-4-tert.-butylsulfonyl-5-nitro-benzamid;
mit 2-Methyl-4-isopropylsulfonyl-5-vitro-benzoesäurechlorid das N-Diaminomethylen-2-methyl-4-isopropylsulfonyl-5-nitro-benzamid; mit 2-Methyl-4-hexylsulfonyl-5-nitro-benzoesäurechlorid das N-Diaminomethylen-2 -methyt-4-hexylsulfonyl-5-nitro-benzamid;
mit 2-Methyl-4-cyclopropylsulfonyl-5-nitro-benzoesäurechlorid das N-Diaminomethylen-2-methyl-4-cyclopropylsulfonyl-5-nitrobenzamid;
mit 2-Methyl-4-cyclopentylsulfonyl-5-nitro-benzoesäurechlorid das N-Diaminomethylen-2-methyl-4-cyclopentylsulfonyl-5-nitrobenzamid;
mit 2-Methyl-4-cyclohexylsulfonyl-5-nitro-benzoesäurechlorid das N-Diaminomethylen-2-methyl-4-cyclohexylsulfonyl-5-nitro-benzamid;
mit 2-Methyl-4-(2-methylcyclopentylsulfonyl)-5-nitro-benzoesäurechlorid das N-Diaminomethylen-2-methyl-4-(2-methylcyclopentylsulfonyl)-5-nitro-benzamid;
mit 2-Methyl-4-cyclobutylsulfonyl-5-nitro-benzoesäurechlorid das N-Diaminomethylen-2-methyl-4-cyclobutylsulfonyl-5-nitro-benzamid;
mit 2-Methyl-4-cycioheptylsulfonyl-5-nitro-benzoesäurechlorid das N-Diaminomethylen-2-methyl-4-cycloheptylsulfonyl-5-nitrobenzamid;
mit 2-Ethyl-4-phenylsulfonyl-5-nitro-benzoesäurechlorid das N-Diaminomethylen-2-ethyi-4-phenylsulfonyl-5-nitro-benzamid;
mit 2-Ethyl-4,5-di-(methylsulfonyl)-benzoesäurechlorid das N-Diaminomethylen-2-ethyl-4-methylsulfonyl-5-nitro-benzamid;
mit 2-Ethyl-4-ethylsulfonyl-5-nitro-benzoesäurechlorid das N-Diaminomethylen-2-ethyl-4-ethylsulfonyl-5-nitro-benzamid;
mit 2-Ethyl-4-propylsulfonyl-5-nitro-benzoesäurechlorid das N-Diaminomethylen-2-ethyl-4-propylsulfonyl-5-nitro-benzamid;
mit 2-Ethyl-4-butylsulfonyl-5-nitro-benzoesäurechlorid das N-Diaminomethylen-2-ethyl-4-butylsulfonyl-5-nitro-benzamid;
mit 2-Ethyl-4-(2-butylsulfonyl)-5-nitro-benzoesäurechlorid das N-Diaminomethylen-2-ethyl-4-(2-butylsulfonyl)-5-nitro-benzamid;
mit 2-Ethyl-4-pentylsulfonyl-5-nitro-benzoesäurechlorid das N-Diaminomethylen-2-ethyl-4-pentylsulfonyl-5-nitro-benzamid;
mit 2-Ethyl-4-(2-pentylsulfonyl)-5-nitro-benzoesäurechlorid das N-Diaminomethylen-2-ethyl-4-(2-pentylsulfonyl)-5-nitro-benzamid;
mit 2-Ethyl-4-(3-pentylsulfonyl)-5-nitro-benzoesäurechlorid das N-Diaminomethylen-2-ethyl-4-(3-pentylsulfonyl)-5-nttro-benzamid;
mit 2-Ethyl-4-tert.-butylsulfonyl-5-nitro-benzoesäurechlorid das N-Diaminomethylen-2-ethyl-4-tert.-butylsulfonyl-5-vitro-benzamid;
mit 2-Ethyl-4-isopropylsulfonyl-5-nitro-benzoesäürechlorid das N-Diaminomethylen-2-ethyl-4-isopropylsulfonyl-5-nitro-benzamid;
mit 2-Ethyl-4-hexylsulfonyl-5-nitro-benzoesäurechlorid das N-Diaminomethylen-2-ethyl-4-hexylsulfonyl-5-nitro-benzamid;
mit 2-Ethyl-4-cyclopropylsulfonyl-5-nitro-benzoesäurechlorid das N-Diaminomethylen-2-ethyl-4-cyclopropylsulfonyl-5-nitro-benzamid;
mit 2-Ethyl-4-cyclopentylsulfonyl-5-nitro-benzoesäurechlorid das N-Diaminomethylen-2-ethyl-4-cyclopentylsulfonyl-5-nitro-benzamid;
mit 2-Ethyl-4-cyclohexylsulfonyl-5-nitro-benzoesäurechlorid das N-Diaminomethylen-2-ethyl-4-cyclohexylsulfonyl-5-nitro-benzamid;
mit 2-Ethyl-4-(2-methylcyclopentylsulfonyl)-5-vitro-benzoesäurechlorid das N-Diaminomethylen-2-ethyl-4-(2-methylcyclopentylsulfonyl)-5-nitrobenzamid;
mit 2-Ethyl-4-cyclobutylsulfonyl-5-nitro-benzoesäurechlorid das N-Diaminomethylen-2-ethyl-4-cyclobutylsulfonyl-5-nitro-benzamid;
mit 2-Ethyl-4-cycloheptylsulfonyl-5-vitro-benzoesäurechlorid das N-Diaminomethylen-2-ethyl-4-cycloheptylsulfonyl-5-nitro-benzamid.

### Beispiel 3

Analog Beispiel 1 erhält man durch Umsetzung von 1,8 g 2-Methyl-4-methylsulfonyl-5-trifluormethyl-benzoesäurechlorid [erhältlich durch Umsetzung von 2-Methyl-4-brom-5-trifluormethyl-benzoesäuremethylester mit Methylthio-zinkchlorid in Gegenwart von Pd-(ll)-[1,1'-bis-(diphenylphosphino)-ferrocen]-chlorid und Cul, anschließende Oxidation mit H₂O₂, Verseifung und Überführung ins Säurechlorid z.B. mit SOCl₂] mit 1,5 g Guanidin in Methanol das N-Diaminomethylen-2-methyl-4-(methylsulfonyl)-5-trifluormethyl-benzamid.

Analog erhält man durch Umsetzung von Guanidin
mit 2-Methyl-4-phenylsulfonyl-5-trifluormethyl-benzoesäurechlorid das N-Diaminomethylen-2-methyl-4-phenylsulfonyl-5-trifluormethylbenzamid;
mit 2-Methyl-4-ethylsulfonyl-5-trifluormethyl-benzoesäurechlorid das N-Diaminomethylen-2-methyl-4-ethylsulfonyl-5-trifluormethylbenzamid;
mit 2-Methyl-4-propylsulfonyl-5-trifluormethyl-benzoesäurechlorid das N-Diaminomethylen-2-methyl-4-propylsulfonyl-5-trifluormethylbenzamid;
mit 2-Methyl-4-butylsulfonyl-5-trifluormethyl-benzoesäurechlorid das N-Diaminomethylen-2-methyl-4-butylsulfonyl-5-trifluormethylbenzamid;
mit 2-Methyl-4-(2-butylsulfonyl)-5-trifluormethyl-benzoesäurechlorid das N-Diaminomethylen-2-methyl-4-(2-butylsulfonyl)-5-trifluormethylbenzamid;
mit 2-Methyl-4-pentylsulfonyl-5-trifluormethyl-benzoesäurechlorid das N-Diaminomethylen-2-methyl-4-pentylsulfonyl-5-trifluormethylbenzamid;
mit 2-Methyl-4-(2-pentylsulfonyl)-5-trifluormethyl-benzoesäurechlorid das N-Diaminomethylen-2-methyl-4-(2-pentylsulfonyl)-5-trifluormethylbenzamid;
mit 2-Methyl-4-(3-pentylsulfonyl)-5-trifluormethyl-benzoesäurechlorid das N-Diaminomethylen-2-methyl-4-(3-pentylsulfonyl)-5-trifluormethyl-benzamid;
mit 2-Methyl-4-tert.-butylsulfonyl-5-trifluormethyl-benzoesäurechlorid das N-Diaminomethylen-2-methyl-4-tert.-butylsulfonyl-5-trifluormethylbenzamid;
mit 2-Methyl-4-isopropylsulfonyl-5-trifluormethyl-benzoesäurechlorid das N -Diaminomethylen-2-methyl-4-isopropylsulfonyl-5-trifluormethylbenzamid;
mit 2-Methyl-4-hexylsulfonyl-5-trifluormethyl-benzoesäurechlorid das N -Diaminomethylen-2-methyl-4-hexylsulfonyl-5-trifluormethylbenzamid;
mit 2-Methyl-4-cyclopropylsulfonyl-5-trifluormethyl-benzoesäurechlorid das N -Diaminomethylen-2-methyl-4-cyclopropylsulfonyl-5-trifluormethylbenzamid;
mit 2-Methyl-4-cyclopentylsulfonyl-5-trifluormethyl-benzoesäurechlorid das N-Diaminomethylen-2-methyl-4-cyclopentylsulfonyl-5-trifluormethylbenzamid;
mit 2-Methyl 2-Methyl-4-cyclohexylsulfonyl-5-trifluormethyl-benzoesäurechlorid das N-Diaminomethylen-2-methyl-4-cyclohexylsulfonyt-5-trifluormethylbenzamid;
mit 2-Methyl-4-(2-methylcyclopentylsulfonyl)-5-trifluormethyl-benzoesäurechlorid das N-Diaminomethylen-2-methyl-4-(2-methylcyclopentylsulfonyl)-5-trifluormethyl-benzamid;
mit 2-Methyl-4-cyclobutylsulfonyl-5-trifluormethyl-benzoesäurechlorid das N-Diaminomethylen-2-methyl-4-cyclobutylsulfonyl-5-trifluormethylbenzamid;
mit 2-Methyl-4-cycloheptylsulfonyl-5-trifluormethyl-benzoesäurechlorid das N-Diaminomethylen-2-methyl-4-cycloheptylsulfonyl-5-trifluormethylbenzamid;
mit 2-Ethyl-4-phenylsulfonyl-5-trifluormethyl-benzoesäurechlorid das N-Diaminomethylen-2-ethyl-4-phenylsulfonyl-5-trifluormethylbenzamid;
mit 2-Ethyl-4,5-di-(methylsulfonyl)-benzoesäurechlorid das N-Diaminomethylen-2-ethyl-4-methylsulfonyl-5-trifluormethylbenzamid;
mit 2-Ethyl-4-ethylsulfonyl-5-trifluormethyl-benzoesäurechlorid das N-Diaminomethylen-2-ethyl-4-ethyisulfonyt-5-trifluormethyl-benzamid;
mit 2-Ethyl-4-propylsulfonyl-5-trifluormethyl-benzoesäurechlorid das N-Diaminomethylen-2-ethyl-4-propylsulfonyl-5-trifluormethylbenzamid;
mit 2-Ethyl-4-butylsulfonyl-5-trifluormethyl-benzoesäurechlorid das N-Diaminomethylen-2-ethyl-4-butylsulfonyl-5-trifluormethylbenzamid;
mit 2-Ethyl-4-(2-butylsulfonyl)-5-trifluormethyl-benzoesäurechlorid das N-Diaminomethylen-2-ethyl-4-(2-butylsulfonyl)-5-trifluormethytbenzamid;
mit 2-Ethyl-4-pentylsulfonyl-5-trifluormethyl-benzoesäurechtorid das N-Diaminomethylen-2-ethyl-4-pentylsulfonyl-5-trifluormethylbenzamid;
mit 2-Ethyl-4-(2-pentylsulfonyl)-5-trifluormethyl-benzoesäurechlorid das N-Diaminomethylen-2-ethyl-4-(2-pentylsulfonyl)-5-trifluormethylbenzamid;
mit 2-Ethyl-4-(3-pentylsulfonyl)-5-trifluormethyl-benzoesäurechlorid das N-Diaminomethylen-2-ethyl-4-(3-pentylsulfonyl)-5-trifluormethylbenzamid;
mit 2-Ethyl-4-tert.-butylsulfonyl-5-trifluormethyl-benzoesäurechlorid das N -Diaminomethylen-2-ethyl-4-tert.-butylsulfonyl-5-trifluormethylbenzamid;
mit 2-Ethyl-4-isopropylsulfonyl-5-trifluormethyl-benzoesäurechlorid das N-Diaminomethylen-2-ethyl-4-isopropylsulfonyl-5-trifluormethylbenzamid;
mit 2-Ethyl-4-hexylsulfonyl-5-trifluormethyl-benzoesäurechlorid das N-Diaminomethylen-2-ethyl-4-hexylsulfonyl-5-trifluormethylbenzamid;
mit 2-Ethyl-4-cyclopropylsulfonyl-5-trifluormethyl-benzoesäurechlorid das N-Diaminomethylen-2-ethyl-4-cyclopropylsulfonyl-5-trifluormethylbenzamid;
mit 2-Ethyl-4-cyclopentylsulfonyl-5-trifluormethyl-benzoesäurechlorid das N-Diaminomethylen-2-ethyl-4-cyclopentylsulfonyl-5-trifluormethylbenzamid;
mit 2-Ethyl-4-cyclohexylsulfonyl-5-trifluormethyl-benzoesäurechlorid das N-Diaminomethylen-2-ethyt-4-cyclohexytsutfonyl-5-trifluormethylbenzamid;
mit 2-Ethyl-4-(2-methylcyclopentylsulfonyl)-5-trifluormethylbenzoesäurechlorid das N-Diaminomethylen-2-ethyl-4-(2-methylcyclopentylsulfonyl)-5-trifluormethyl-benzamid;
mit 2-Ethyl-4-cyclobutylsulfonyl-5-trifluormethyl-benzoesäurechlorid das N-Diaminomethylen-2-ethyl-4-cyclobutylsulfonyl-5-trifluormethylbenzamid;
mit 2-Ethyl-4 -cycloheptylsulfonyl-5-trifluormethyl-benzoesäurechlorid das N-Diaminomethylen-2-ethyl-4-cycloheptylsulfonyl-5-trifluormethylbenzamid.

### Beispiel 4

700 mg N-Diaminomethylen-2-methyl-4,5-di-(methylsulfonyl)-benzamid [erhältlich nach Bsp. 1] werden in 50 ml Wasser suspendiert und unter Rühren mit 1,8 ml 1 n HCI versetzt. Nach Filtration und Lyophilisation erhält man erhält N-Diaminomethylen-2-methyl-4,5-di-(methylsulfonyl)-benzamid; Hydrochlorid, F. > 240°.

Analog erhält man aus den freien Basen die folgenden Hydrochloride:
N-Diaminomethylen-2-methyl-4-phenylsulfonyl-5-methylsulfonylbenzamid, Hydrochlorid, F. > 260°;
N -Diaminomethylen-2-methyl-4-cyclopropylsulfonyl-5-methylsulfonylbenzamid, Hydrochlorid;
N-Diaminomethylen-2-methyl-4-ethylsulfonyl-5-methylsulfonyl-benzamid, Hydrochlorid;
N-Diaminomethylen-2-methyl-4-propylsulfonyl-5-methylsulfonyl-benzamid, Hydrochlorid;
N-Diaminomethylen-2-methyl-4-cyclopentylsulfonyl-5-methylsulfonylbenzamid, Hydrochlorid;
N-Diaminomethylen-2-methyl-4-isopropylsulfonyl-5-methylsulfonylbenzamid, Hydrochlorid;
N-Diaminomethylen-2-methyl-4-isopropylsulfonyl-5-nitro-benzamid, Hydrochlorid;
N-Diaminomethylen-2-methyl-4-methylsulfonyl-5-nitro-benzamid, Hydrochlorid;
N-Diaminomethylen-2-methyl-4-methylsulfonyl-5-trifluormethyl-benzamid, Hydrochlorid;
N-Diaminomethylen-2-methyl-4-Isopropylsulfonyl-5-trifluormethylbenzamid, Hydrochlorid;
N-Diaminomethylen-2-ethyl-4,5-di-(methylsulfonyl)-benzamid, Hydrochlorid;
N-Diaminomethylen-2-ethyl-4-methylsulfonyl-5-nitro-benzamid, Hydrochlorid;
N-Diaminomethylen-2-ethyl-4-methylsulfonyl-5-trifluormethyl-benzamid, Hydrochlorid;
N-Diaminomethylen-2-ethyl-4-cyclopropylsulfonyl-5-m ethylsuilonylbenzamid, Hydrochlorid;
N-Diaminomethylen-2-ethyl-4-cyclopentylsulfonyl-5-methylsulfonylbenzamid, Hydrochlorid;
N-Diaminomethylen-2-ethyl-4-butyl-5-methylsulfonyl-benzamid, Hydrochlorid.

### Beispiel 5

Eine Suspension von 1 g N-Diaminomethylen-2-methyl-4-chlor-5-methylsulfonylbenzamid [erhältlich durch Umsetzung von 2-Methyl-4-chlor-5-methylsulfonyl-benzoylchlorid mit Guanidin in Gegenwart von Triethylamin], 7 ml Thiophenol und 6g K₂CO₃ wird 90 min auf 180° erhitzt. Es wird in Wasser gelöst, mit Essigsäureethylester extrahiert und eingedampft. Man erhält das N-Diaminomethylen-2-methyl-4-(2-methylphenylthio)-5-methylsulfonyl-benzamid, woraus nach Behandlung mit 1,5 g Natriumperborat in 30 ml Eisessig über einen Zeitraum von 2 Stunden bei 60° im Wasserbad nach üblicher Aufarbeitung das N-Diamino-methylen-2-methyl-4-(2-methylphenylsulfonyl)-5-methylsulfonyl-benzamid erhalten wird.

### Beispiel 6

Analog Beispiel 5 erhält man durch Oxidation der entsprechenden Thioverbindungen, die auch durch Umsetzung von N-Diaminomethylen-2-alkyl-4-arylthio (bzw. 4-alkylthio)-5-methylsulfonyl-benzoesäurechlorid mit Guanidin in Gegenwart von Base hergestellt werden können, die folgenden Phenyl-substituierten N-Diaminomethylen-2-alkyl-4-phenylsulfonyl-5-methylsulfonylbenzamide:
aus N-Diaminomethylen-2-methyl-4-(3-methylphenylthio)-5-methylsulfonyl-benzamid das N-Diaminomethylen-2-methyl-4-(3-methylphenylsulfonyl)-5-methylsulfonyl-benzamid;
aus N-Diaminomethylen-2-methyl-4-(4-methylphenylthio)-5-methylsulfonyl-benzamid das N-Diaminomethylen-2-methyl-4-(4-methylphenylsulfonyl)-5-methylsulfonyl-benzamid;
aus N-Diaminomethylen-2-methyl-4-(2,4-dimethylphenylthio)-5-methylsulfonyl-benzamid das N-Diaminomethylen-2-methyl-4-(2,4-dimethylphenylsulfonyl)-5-methylsulfonyl-benzamid;
aus N-Diaminomethylen-2-methyl-4-(2-chlorphenylthio)-5-methylsulfonylbenzamid das N-Diaminomethylen-2-methyl-4-(2-chlorphenylsulfonyl)-5-methylsulfonyl-benzamid;
aus N-Diaminomethylen-2-methyl-4-(3-chlorphenylthio)-5-methylsulfonylbenzamid das N-Diaminomethylen-2-methyl-4-(3-chlorphenylsulfonyl)-5-methylsulfonyl-benzamid;
aus N-Diaminomethylen-2-methyl-4-(4-chlorphenylthio)-5-methylsulfonylbenzamid das N-Diaminomethylen-2-methyl-4-(4-chlorphenylsulfonyl)-5-methylsulfonyl-benzamid;
aus N-Diaminomethylen-2-methyl-4-(2-bromphenylthio)-5-methylsulfonylbenzamid das N-Diaminomethylen-2-methyl-4-(2-bromphenylsulfonyl)-5-methylsulfonyl-benzamid;
aus N-Diaminomethylen-2-methyl-4-(4-bromphenylthio)-5-methylsulfonylbenzamid das N-Diaminomethylen-2-methyl-4-(4-bromphenylsulfonyl)-5-methylsulfonyl-benzamid;
aus N-Diaminomethylen-2-methyl-4-(2-trifluormethyl-phenylthio)-5-methylsulfonyl-benzamid das N-Diaminomethylen-2-methyl-4-(2-trifluormethyl-phenyfsulfonyl)-5-methylsulfonyl-benzamid;
aus N-Diaminomethylen-2-methyl-4-(3-trifluormethyl-phenylthio)-5-methylsulfonyl-benzamid das N-Diaminomethylen-2-methyl-4-(3-trifluormethyl-phenylsulfonyl)-5-methylsulfonyl-benzamid;
aus N-Diaminomethylen-2-methyl-4-(4-trifluormethyl-phenylthio)-5-methylsulfonyl-benzamid das N-Diaminomethylen-2-methyl-4-(4-trifluormethyl-phenylsulfonyl)-5-methylsulfonyl-benzamid;
aus N-Diaminomethylen-2-ethyl-4-(2-methylphenylthio)-5-methylsulfonylbenzamid das N-Diaminomethylen-2-ethyl-4-(2-methylphenylsulfonyl)-5-methylsulfonyl-benzamid;
aus N-Diaminomethylen-2-ethyl-4-(3-methylphenylthio)-5-methylsulfonylbenzamid das N-Diaminomethylen-2-ethyl-4-(3-methylphenylsulfonyl)-5-methylsulfonyl-benzamid;
aus N-Diaminomethylen-2-ethyl-4-(4-methylphenylthio)-5-methylsulfonylbenzamid das N-Diaminomethylen-2-ethyl-4-(4-methylphenylsulfonyl)-5-methylsulfonyl-benzamid;
aus N-Diaminomethylen-2-ethyl-4-(2,4-dimethylphenylthio)-5-methylsulfonyl-benzamid das N-Diaminomethylen-2-ethyl-4-(2,4-dimethylphenylsulfonyl)-5-methylsulfonyl-benzamid;
aus N-Diaminomethylen-2-ethyl-4-(2-chlorphenylthio)-5-methylsulfonylbenzamid das N-Diaminomethylen-2-ethyl-4-(2-chlorphenylsulfonyl)-5-methylsulfonyl-benzamid;
aus N-Diaminomethylen-2-ethyl-4-(3-chlorphenylthio)-5-methylsulfonylbenzamid das N-Diaminomethylen-2-ethyl-4-(3-chlorphenylsulfonyl)-5-methylsulfonyl-benzamid;
aus N-Diaminomethylen-2-ethyl-4-(4-chlorphenylthio)-5-methylsulfonylbenzamid das N-Diaminomethylen-2-ethyl-4-(4-chlorphenylsulfonyl)-5-methylsulfonyl-benzamid;
aus N-Diaminomethylen-2-ethyl-4-(2-bromphenylthio)-5-methylsulfonylbenzamid das N-Diaminomethylen-2-ethyl-4-(2-bromphenylsulfonyl)-5-methylsulfonyl-benzamid;
aus N-Diaminomethylen-2-ethyl-4-(4-bromphenylthio)-5-methylsuffonylbenzamid das N-Diaminomethylen-2-ethyl-4-(4-bromphenylsulfonyl)-5-methylsulfonyl-benzamid;
aus N-Diaminomethylen-2-ethyl-4-(2-trifluormethyl-phenylthio)-5-methylsulfonyl-benzamid das N-Diaminomethylen-2-ethyl-4-(2-trifluormethyl-phenylsulfonyl)-5-methylsulfonyl-benzamid;
aus N-Diaminomethylen-2-ethyl-4-(3-trifluormethyl-phenylthio)-5-methylsulfonyl-benzamid das N-Diaminomethylen-2-ethyl-4-(3-trifluormethyl-phenylsulfonyl)-5-methylsulfonyl-benzamid;
aus N-Diaminomethylen-2-ethyl-4-(4-trifluormethyl-phenylthio)-5-methylsulfonyl-benzamid das N-Diaminomethylen-2-ethyl-4-(4-trifluormethyl-phenylsulfonyl)-5-methylsulfonyl-benzamid.

### Beispiel 7

Analog Beispiel 1 erhält man durch Umsetzung von 1,3 g 2-Methyl-4-methylsulfinyl-5-methylsulfonyl-benzoesäurechlorid [erhältlich durch Umsetzung von 2-Methyl-4-chlor-5-methylsulfonyl-benzoesäure-mit Namethanthiolat, anschließende partielle Oxidation mit Wasserstoffperoxid und Überführung ins Säurechlorid z.B. mit SOCl₂] mit Guanidin nach üblicher Aufarbeitung und Umkristallisieren aus Diethylether das N-Diaminomethylen-2-methyl-4-methylsulfinyl-5-methylsulfonyl-benzamid.

Analog erhält man durch Umsetzung von Guanidin
mit 2-Methyl-4-phenylsulfinyl-5-methylsulfonyl-benzoesäurechlorid das N-Diaminomethylen-2-methyl-4-phenylsulfinyl-5-methylsulfonylbenzamid, F. 235-237°;
mit 2-Methyl-4-ethylsulfinyl-5-methylsulfonyl-benzoesäurechlorid das N-Diaminomethylen-2-methyl-4-ethylsulfinyl-5-methylsulfonylbenzamid;
mit 2-Methyl-4-propylsulfinyl-5-methylsulfonyl-benzoesäurechlorid das N-Diaminomethylen-2-methyl-4-propylsulfinyl-5-methylsulfonylbenzamid;
mit 2-Methyl-4 -butylsulfinyl-5-methylsulfonyl-benzoesäurechlorid das N-Diaminomethylen-2-methyl-4-butylsulfinyl-5-methylsulfonylbenzamid;
mit 2-Methyl-4- (2-butylsulfinyl)-5-methylsulfonyl-benzoesäurechlorid das N-Diaminomethylen-2-methyl-4-(2-butylsulfinyl)-5-methylsulfonylbenzamid;
mit 2-Methyl-4-pentylsulfinyl-5-methylsulfonyl-benzoesäurechlorid das N-Diaminomethylen-2-methyl-4-pentylsulfinyl-5-methylsulfonylbenzamid;
mit 2-Methyl-4-(2-pentylsulfinyl)-5-methylsulfonyl-benzoesäurechlorid das N-Diaminomethylen-2-methyl-4-'(2-pentylsutfinyl)-5-methylsulfonylbenzamid;
mit 2-Methyl-4-(3-pentylsulfinyl)-5-methylsulfonyl-benzoesäurechlorid das N-Diaminomethylen-2-methyl-4-(3-pentylsulfinyl)-5-methylsulfonylbenzamid;
mit 2-Methyl-4-tert.-butylsulfinyl-5-methylsulfonyl-benzoesäurechlorid das N-Diaminomethylen-2-methyl-4-tert.-butylsulfinyl-5-methylsulfonylbenzamid;
mit 2-Methyl-4-isopropylsulfinyl-5-methylsulfonyl-benzoesäurechlorid das N-Diaminomethylen-2 -methyl-4-isopropylsulflnyl-5-methylsulfonylbenzamid;
mit 2-Methyl-4-hexylsulfinyl-5-methylsulfonyl-benzoesäurechlorid das N-Diaminomethylen-2-methyl-4-hexylsulfinyl-5-methylsulfonylbenzamid;
mit 2-Methyl-4-cyclopropylsulfinyl-5-methylsulfonyl-benzoesäurechlorid das N-Diaminomethylen-2-methyl-4-cyclopropylsulfinyl-5-methylsulfonylbenzamid:
mit 2-Methyl-4-cyclopentylsulfinyl-5-methylsulfonyl-benzoesäurechlorid das N-Diaminomethylen-2-methyl-4-cyclopentylsulfinyl-5-methylsulfonylbenzamid;
mit 2-Methyl-4-cyclohexylsulfinyl-5-methylsulfonyl-benzoesäurechlorid das N-Diaminomethylen-2-methyl-4-cyclohexylsulfinyl-5-methylsulfonylbenzamid;
mit 2-Methyl-4-(2-methylcyclopentylsulfinyl)-5-methylsulfonyl-benzoesäurechlorid das N-Diaminomethylen-2-methyl-4-(2-methylcyclopentylsulfinyl)-5-methylsulfonyl-benzamid;
mit 2-Methyl-4-cyclobutylsulfinyl-5-methylsulfonyl-benzoesäurechlorid das N-Diaminomethylen-2-methyl-4-cyclobutylsulfinyl-5-methylsulfonylbenzamid;
mit 2-Methyl-4-cycloheptylsulfinyl-5-methylsulfonyl-benzoesäurechlorid das N-Diaminomethylen-2-methyl-4-cycloheptylsulfinyl-5-methylsulfonylbenzamid;
mit 2-Ethyl-4-phenylsulfinyl-5-methylsulfonyl-benzoesäurechlorid das N-Diaminomethylen-2-ethyl-4-phenylsulfinyl-5-methylsulfonylbenzamid;
mit 2-Ethyl-4,5-di-(methylsulfinyl)-benzoesäurechlorid das N-Diaminomethylen-2-ethyl-4,5-di-(methylsulfinyl)-benzamid;
mit 2-Ethyl-4-ethylsulfinyl-5-methylsulfonyl-benzoesäurechlorid das N-Diaminomethylen-2-ethyl-4-ethylsulfinyl-5-methylsulfonylbenzamid;
mit 2-Ethyl-4-propylsulfinyl-5-methylsulfonyl-benzoesäurechlorid das N-Diaminomethylen-2-ethyl-4-propylsulfinyl-5-methylsulfonylbenzamid;
mit 2-Ethyl-4-butylsulfinyl-5-methylsulfonyl-benzoesäurechlorid das N-Diaminomethylen-2-ethyl-4-butylsulfinyl-5-methylsulfonylbenzamid;
mit 2-Ethyl-4-(2-butylsulfinyl)-5-methylsulfonyl-benzoesäurechlorid das N-Diaminomethylen-2-ethyl-4-(2-butylsulfinyl)-5-methylsulfonyl-benzamid;
mit 2-Ethyl-4-pentylsulfinyl-5-methylsulfonyl-benzoesäurechlorid das N-Diaminomethylen-2-ethyl-4-pentylsulfinyl-5-methylsulfonylbenzamid;
mit 2-Ethyl-4-(2-pentylsulfinyl)-5-methylsulfonyl-benzoesäurechlorid das N-Diaminomethylen-2-ethyl-4-(2-pentylsulfinyl)-5-methylsulfonylbenzamid;
mit 2-Ethyl-4-(3-pentylsulfinyl)-5-methylsulfonyl-benzoesäurechlorid das N-Diaminomethylen-2-ethyl-4-(3-pentylsulfinyl)-5-methylsulfonylbenzamid;
mit 2-Ethyl-4-tert.-butylsuffinyl-5-methylsulfonyl-benzoesäurechiorid das N-Diaminomethylen-2-ethyl-4-tert.-butylsulfinyl-5-methylsulfonylbenzamid;
mit 2-Ethyl-4-isopropylsulfinyl-5-methylsulfonyl-benzoesäurechlorid das N-Diaminomethylen-2-ethyl-4-isopropylsulfinyl-5-methylsulfonylbenzamid;
mit 2-Ethyl-4-hexylsulfinyl-5-methylsulfonyi-benzoesäurechlorid das N-Diaminomethylen-2-ethyl-4-hexylsulfinyl-5-methylsulfonylbenzamid;
mit 2-Ethyl-4-cyclopropylsulfinyl-5-methylsulfonyl-benzoesäurechlorid das N-Diaminomethylen-2-ethyl-4-cyclopropylsulfinyl-5-mefhylsulfonylbenzamid;
mit 2-Ethyl-4-cyclopentylsulfinyl-5-methylsulfonyl-benzoesäurechlorid das N-Diaminomethylen-2-ethyl-4-cyclopentylsulfinyl-5-methylsulfonylbenzamid;
mit 2-Ethyl-4-cyclohexylsulfinyl-5-methylsulfonyl-benzoesäurechlorid das N-Diaminomethylen-2-ethyl-4-cyclohexylsulfinyl-5-methylsulfonylbenzamid;
mit 2-Ethyl-4-(2-methylcyclopentylsulfinyl)-5-methylsulfonylbenzoesäurechlorid das N-Diaminomethylen-2-ethyl-4-(2-methylcyclopentylsulfinyl)-5-methylsulfonyl-benzamid;
mit 2-Ethyl-4-cyclobutylsulfinyl-5-methylsulfonyl-benzoesäurechlorid das N-Diaminornethylen-2-ethyl-4-cyclobutylsulfinyl-5-methylsulfonylbenzamid;
mit 2-Ethyl-4-cycloheptylsulfinyl-5-methylsulfonyl-benzoesäurechlorid das N-Diaminomethylen-2-ethyl-4-cycloheptylsulfinyl-5-methylsulfonylbenzamid.

### Beispiel 8

Zu einer Suspension von 1 g N-Diaminomethylen-2-methyl-4-brom-5-nitro-benzamid [erhältlich durch Umsetzung von 2-Methyl-4-brom-5-methylsulfonyl-benzoylchlorid mit Guanidin in Gegenwart von Triethylamin] in 70 ml THF fügt man nacheinander 20 mg Pd-(ll)-[1,1'-bis-(diphenyl-phosphin)-ferrocen]-chlorid, 10 mg Cul sowie 2 Equivalente Phenylsulfinyl-zinkchlorid, gelöst in 10 ml THF hinzu und rührt 2 Stunden bei Raumtemperatur. Anschließend erhält man nach Filtration, Entfernung des Lösungsmittels und üblicher Aufarbeitung das N-Diaminomethylen-2-methyl-4-phenylsulfinyl-5-nitro-benzamid, woraus nach Behandlung mit verdünnter wäßriger HCI-Lösung und Gefriertrocknung das entsprechende Hydrochlorid erhalten wird.

### Beispiel 9

Analog Beispiel 6 erhält man durch Oxidation der entsprechenden Thioverbindungen, die wie zuvor beschrieben, durch Umsetzung von 2-Alkyl-4-arylthio-5-nitro-benzoesäureclorid mit Guanidin in Gegenwart von Base hergestellt werden können, die folgenden Aryl-substituierten N-Diaminomethylen-2-alkyl-4-arylsulfonyl-5-nitro-benzamide:
aus N-Diaminomethylen-2-methyl-4-(3-methylphenylthio)-5-nitrobenzamid das N-Diaminomethylen-2-methyl-4-(3-methylphenylsulfonyl)-5-nitrobenzamid;
aus N-Diaminomethylen-2-methyl-4-(4-methylphenylthio)-5-nitro-benzamid das N-Diaminomethylen-2-methyl-4-(4-methylphenylsulfonyl)-5-nitrobenzamid;
aus N-Diaminomethylen-2-methyl-4-(2,4-dimethylphenylthio)-5-nitrobenzamid das N-Diaminomethylen-2-methyl-4-(2,4-dimethylphenylsulfonyl)-5-nitrobenzamid;
aus N-Diaminomethylen-2-methyl-4-(2-chlorphenylthio)-5-nitro-benzamid das N-Diaminomethylen-2-methyl-4-(2-chlorphenylsulfonyl)-5-nitrobenzamid;
aus N-Diaminomethylen-2-methyl-4-(3-chlorphenylthio)-5-nitro-benzamid das N-Diaminomethylen-2-methyl-4-(3-chlorphenylsulfonyl)-5-nitrobenzamid:
aus N-Diaminomethylen-2-methyl-4-(4-chlorphenylthio)-5-nitro-benzamid das N-Diaminomethylen-2-methyl-4-(4-chlorphenylsulfonyl)-5-nitrobenzamid;
aus N-Diaminomethylen-2-methyl-4-(2-bromphenylthio)-5-nitro-benzamid das N-Diaminomethylen-2-methyl-4-(2-bromphenylsulfonyl)-5-nitrobenzamid;
aus N-Diaminomethylen-2-methyl-4-(4-bromphenylthio)-5-nitro-benzamid das N-Diaminomethylen-2-methyl-4-(4-bromphenylsulfonyl)-5-nitrobenzamid;
aus N-Diaminomethylen-2-methyl-4-(2-trifluormethyl-phenylthio)-5-nitrobenzamid das N-Diaminomethylen-2-methyl-4-(2-trifluormethyl-phenylsulfonyl)-5-nitro-benzamid;
aus N-Diaminomethylen-2-methyl-4-(3-trifluormethyl-phenylthio)-5-nitrobenzamid das N-Diaminomethylen-2-methyl-4-(3-trifluormethyl-phenylsulfonyl)-5-nitro-benzamid;
aus N-Diaminomethylen-2-methyl-4-(4-trifluormethyl-phenylthio)-5-nitrobenzamid das N-Diaminomethylen-2-methyl-4-(4-trifluormethyl-phenylsulfonyl)-5-nitro-benzamid;
aus N-Diaminomethylen-2-ethyl-4-(2-methylphenylthio)-5-nitro-benzamid das N-Diaminomethylen-2-ethyl-4-(2-methylphenylsulfonyl)-5-nitrobenzamid;
aus N-Diaminomethylen-2-ethyl-4-(3-methylphenylthio)-5-nitro-benzamid das N-Diaminomethylen-2-ethyl-4-(3-methylphenylsulfonyl)-5-nitrobenzamid;
aus N-Diaminomethylen-2-ethyl-4-(4-methylphenylthio)-5-vitro-benzamid das N -Diaminomethylen-2-ethyl-4-(4-methylphenylsullonyl)-5-nitrobenzamid;
aus N-Diaminomethylen-2-ethyl-4-(2,4-dimethylphenylthio)-5-nitrobenzamid das N-Diaminomethylen-2-ethyl-4-(2,4-dimethylphenylsulfonyl)-5-nitro-benzamid;
aus N-Diaminomethylen-2-ethyl-4-(2-chlorphenylthio)-5-nitro-benzamid das N-Diaminomethylen-2-ethyl-4-(2-chlorphenylsulfonyl)-5-nitrobenzamid;
aus N-Diaminomethylen-2-ethyl-4-(3-chlorphenylthio)-5-vitro-benzamid das N-Diaminomethylen-2-ethyl-4-(3-chlorphenylsulfonyl)-5-nitrobenzamid;
aus N-Diaminomethylen-2-ethyl-4-(4-chlorphenylthio)-5-nitro-benzamid das N-Diaminomethylen-2-ethyl-4-(4-chlorphenylsulfonyl)-5-nitrobenzamid;
aus N-Diaminomethylen-2-ethyl-4-(2-bromphenyfthio)-5-nitro-benzamid das N-Diaminomethylen-2-ethyl-4-(2-bromphenylsulfonyl)-5-nitrobenzamid;
aus N-Diaminomethylen-2-ethyl-4-(4-bromphenylthio)-5-nitro-benzamid das N-Diaminomethylen-2-ethyl-4-(4-bromphenylsulfonyl)-5-nitrobenzamid;
aus N-Diaminomethylen-2-ethyl-4-(2-trifluormethyl-phenylthio)-5-nitrobenzamid das N-Diaminomethylen-2-ethyl-4-(2-trifluormethyl-phenylsulfonyl)-5-nitro-benzamid;
aus N-Diaminomethylen-2-ethyl-4-(3-trifluormethyl-phenylthio)-5-nitrobenzamid das N-Diaminomethylen-2-ethyl-4-(3-trifluormethyl-phenylsulfonyl)-5-nitro-benzamid;
aus N-Diaminomethylen-2-ethyl-4-(4-trifluormethyl-phenylthio)-5-nitrobenzamid das N-Diaminomethylen-2-ethyl-4-(4-trifluormethyl-phenylsulfonyl)-5-nitro-benzamid.

### Beispiel 10

Analog Beispiel 6 erhält man durch Oxidation der entsprechenden Thioverbindungen, die wie zuvor beschrieben, durch Umsetzung von 2-Alkyl-4-arylthio-5-trifluormethylbenzoesäurechlorid mit Guanidin in Gegenwart von Base hergestellt werden können, die folgenden Phenyl-substituierten N-Diaminomethylen-2-alkyl-4-phenylsulfonyl-5-trifluormethylbenzamide:
aus N-Diaminomethylen-2-methyl-4-(3-methylphenylthio)-5-trifluormethylbenzamid das N-Diaminomethylen-2-methyl-4-(3-methylphenylsulfonyl)-5-trifluormethyl-benzamid;
aus N-Diaminomethylen-2-methyl-4-(4-methylphenylthio)-5-trifluormethyl-benzamid das N-Diaminomethylen-2-methyl-4-(4-methylphenylsulfonyl)-5-trifluormethyl-benzamid;
aus N-Diaminomethylen-2-methyl-4-(2,4-dimethylphenylthio)-5-trifluormethyl-benzamid das N-Diaminomethylen-2-methyl-4-(2,4-dimethylphenylsulfonyl)-5-trifluormethyl-benzamid;
aus N-Dlamlnomethylen-2-methyl-4-(2-chlorphenylthio)-5-trifluormethylbenzamid das N-Diaminomethylen-2-methyl-4-(2-chlorphenylsulfonyl)-5-trifluormethyl-benzamid;
aus N-Diaminomethylen-2-methyl-4-(3-chlorphenylthio)-5-trifluormethylbenzamid das N-Diaminomethylen-2-methyl-4-(3-chlorphenylsulfonyl)-5-trifluormethyl-benzamid;
aus N-Diaminomethylen-2-methyl-4-(4-chlorphenylthio)-5-trifluormethylbenzamid das N-Diaminomethylen-2-methyl-4-(4-chlorphenylsulfonyl)-5-trifluormethyl-benzamid;
aus N-Diaminomethylen-2-methyl-4-(2-bromphenylthio)-5-trifluormethylbenzamid das N-Diaminomethylen-2-methyl-4-(2-bromphenylsulfonyl)-5-trifluormethyl-benzamid;
aus N-Diaminomethylen-2-methyl-4-(4-bromphenylthio)-5-trifluormethylbenzamid das N-Diaminomethylen-2-methyl-4-(4-bromphenylsulfonyl)-5-trifluormethyl-benzamid;
aus N-Diaminomethylen-2-methyl-4-(2-trifluormethyl-phenylthio)-5-trifluormethyl-benzamid das N-Diaminomethylen-2-methyl-4-(2-trifluormethyl-phenylsulfonyl)-5-trifluormethyl-benzamid;
aus N-Diaminomethylen-2-methyl-4-(3-trifluormethyl-phenylthio)-5-trifluormethyl-benzamid das N-Diaminomethylen-2-methyl-4-(3-trifluormethyl-phenylsulfonyl)-5-trifluormethyl-benzamid;
aus N-Diaminomethylen-2-methyl-4-(4-trifluormethyl-phenylthio)-5-trifluormethyl-benzamid das N-Diaminomethylen-2-methyl-4-(4-trifluormethyl-phenylsulfonyl)-5-trifluormethyl-benzamid;
aus N-Diaminomethylen-2-ethyl-4-(2-methylphenylthio)-5-trifluormethylbenzamid das N-Diaminomethylen-2-ethyl-4-(2-methylphenylsulfonyl)-5-trifluormethyl-benzamid;
aus N-Diaminomethylen-2-ethyl-4-(3-methylphenylthio)-5-trifluormethylbenzamid das N-Diaminomethylen-2-ethyl-4-(3-methylphenylsulfonyl)-5-trifluormethyl-benzamid;
aus N-Diaminomethylen-2-ethyl-4-(4-methylphenylthio)-5-trifluormethylbenzamid das N-Diaminomethylen-2-ethyl-4-(4-methylphenylsulfonyl)-5-trifluormethyl-benzamid;
aus N-Diaminomethylen-2-ethyl-4-(2,4-dimethylphenylthio)-5-trifluormethyl-benzamid das N-Diaminomethylen-2-ethyl-4-(2,4-dimethylphenylsulfonyl)-5-trifluormethyl-benzamid;
aus N-Diaminomethylen-2-ethyl-4-(2-chlorphenylthio)-5-trifluormethyl-benzamid das N-Diaminomethylen-2-ethyl-4-(2-chlorphenylsulfonyl)-5-trifluormethyl-benzamid;
aus N-Diaminomethylen-2-ethyl-4-(3-chlorphenylthio)-5-trifluormethylbenzamid das N-Diaminomethylen-2-ethyl-4-(3-chlorphenylsulfonyl)-5-trifluormethyl-benzamid;
aus N-Diaminomethylen-2-ethyl-4-(4-chlorphenylthio)-5-trifluormethylbenzamid das N-Diaminomethylen-2-ethyl-4-(4-chlorphenylsulfonyl)-5-trifluormethyl-benzamid;
aus N-Diaminomethylen-2-ethyl-4-(2-bromphenylthio)-5-trifluormethylbenzamid das N-Diaminomethylen-2-ethyl-4-(2-bromphenylsulfonyl)-5-trifluormethyl-benzamid;
aus N-Diaminomethylen-2-ethyl-4-(4-bromphenylthio)-5-trifluormethylbenzamid das N-Diaminomethylen-2-ethyl-4-(4-bromphenylsulfonyl)-5-trifluormethyl-benzamid;
aus N-Diaminomethylen-2-ethyl-4-(2-trifluormethyl-phenylthio)-5-trifluormethyl-benzamid das N-Diaminomethylen-2-ethyl-4-(2-trifluormethyl-phenylsulfonyl)-5-trifluormethyl-benzamid;
aus N-Diaminomethylen-2-ethyl-4-(3-trifluormethyl-phenylthio)-5-trifluormethyl-benzamid das N-Diaminomethylen-2-ethyl-4-(3-trifluormethyl-phenylsulfonyl)-5-trifluormethyl-benzamid;
aus N-Diaminomethylen-2-ethyl-4-(4-trifluormethyl-phenylthio)-5-trifluormethyl-benzamid das N-Diaminomethylen-2-ethyl-4-(4-trifluormethyl-phenytsulfonyl)-5-trifluormethyl-benzamid.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 31 zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel l, 9,38 g NaH₂PO₄·2 H₂O, 28,48 g Na₂HPO₄·12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. 4-Sulfonyl- oder 4-Sulfinyl-benzoylguanidin-Derivate der Formel l 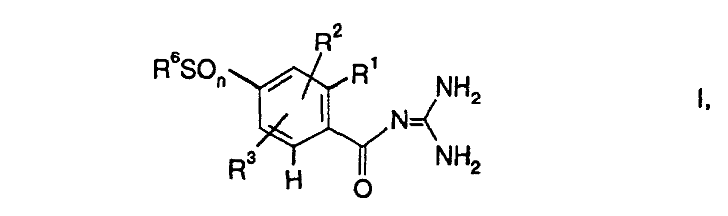 worin
R¹ A, CF₃, CH₂F, CHF₂, C₂F₅, CN, NO₂ oder Hal,
R² -SOₙ-R⁶, -SO₂NR⁴R⁵, NO₂ oder CF₃,
R³ H, Hal, A, OH, OA, CN, NO₂, CF₃, CH₂F, CHF₂, C₂F₅ oder CH₂CF₃,
R⁴ und R⁵ jeweils unabhängig voneinander H, A, Cycloalkyl mit 5 bis 7 C-Atomen, Cycloalkylmethyl mit 4 bis 8 C-Atomen, Ph oder -CH₂-Ph,
oder aber
R⁴ und R⁵ zusammen auch Alkylen mit 4 bis 5 C-Atomen, wobei eine CH₂-Gruppe auch durch O, S, NH, N-A oder N-CH₂-Ph ersetzt sein kann,
R⁶ A, Ph, Het oder C₃-C₇-Cycloalkyl;
Het einen ein- oder zweikernigen gesättigten, ungesättigten oder aromatischen Heterocyclus mit 1 bis 4 N-, O-, und/oder S-Atomen, über N oder C gebunden, der unsubstituiert oder ein-, zwei- oder dreifach durch Hal, SH, SA, NH₂, NHA, NA₂, CF₃, A, OH, OA, CN, NO₂, NHA, NA₂ und/oder Carbonylsauerstoff substituiert sein kann,
A Alkyl mit 1 bis 6C-Atomen,
Hal F, Cl, Br oder l,
Ph unsubstituiertes oder ein-, zwei- oder dreifach durch A, OA, NR⁴R⁵,F, Cl, Br, l oder CF₃ substituiertes Phenyl und
n 1 oder 2,
bedeuten,
sowie deren physiologisch unbedenkliche Salze.

2. (a) N-Diaminomethylen-2-methyl-4,5-di(methylsulfonyl)-benzamid;
(b) N-Diaminomethylen-2-methyl-4-phenylsulfonyl-5-methylsulfonyl-benzamid;
(c) N-Diaminomethylen-2-methyl-4-tert.-butylsulfonyl-5-methylsulfonyl-benzamid;
(d) N-Diaminomethylen-2-ethyl-4,5-di(methylsulfonyl)-benzamid;
(e) N-Diaminomethylen-2-ethyl-4-cyclopentylsulfonyl-benzamid;
(f) N-Diaminomethylen-2-methyl-4-cyclohexylsulfonyl-5-methylsulfonyl-benzamid;
(g) N-Diaminomethylen-2-methyl-4-methylsulfonyl-5-p-chlorphenylsulfonyl-benzamid
gemäß Anspruch 1, sowie deren physiologisch unbedenkliche Salze.

3. Verfahren zur Herstellung von Alkylbenzoylguanidin-Derivaten -Derivaten der Formel I gemäß Anspruch 1 sowie von deren Salzen, **dadurch gekennzeichnet, daß** man eine Verbindung der Formel II 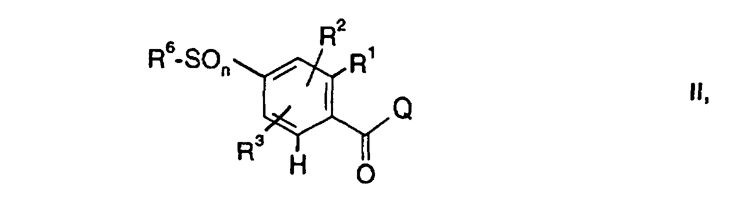 worin R¹, R², R³ und R⁶ sowie n die zuvor angegebenen Bedeutungen haben und
Q Cl, Br, OA, O-CO-A, O-CO-Ph, OH oder eine andere reaktionsfähige veresterte OH-Gruppe bzw. leicht nucleophil substituierbare Abgangsgruppe bedeutet,
mit Guanidin umsetzt, oder daß man ein Benzoylguanidin der Formel lll 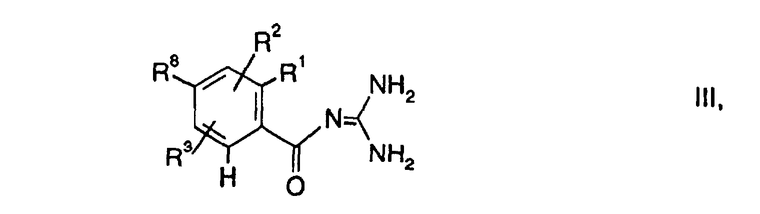 worin R¹, R² und R³ die zuvor angegebenen Bedeutungen besitzen,
und
R⁸ F, Cl, Br, l oder H bedeutet,
mit einer Kohlenwasserstoffverbindung der Formel IV
R⁶-SOₙ-X IV,
worin R⁶ die angegebene Bedeutung besitzt und
X H, Cl, Br oder l bedeutet,
in Gegenwart eines Katalysators, nach vorheriger Metallierung bzw. Transmetallierung, umsetzt,
oder daß man ein Benzoylguandin der Formel V, 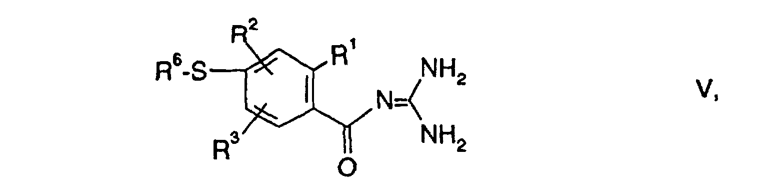 mit einem geeigneten Oxidationsmittel umsetzt,
oder daß man eine sonst der Formel I entsprechende Verbindung, die jedoch anstelle eines oder mehrerer Wasserstoffatome eine oder mehrere reduzierbare Gruppe(n) und/oder eine oder mehrere zusätzliche C-C- und/oder C-N-Bindung(en) enthält, mit einem reduzierenden Mittel behandelt,
oder daß man eine sonst der Formel l entsprechende Verbindung, die jedoch anstelle eines oder mehrerer Wasserstoffatome eine oder mehrere solvolysierbare Gruppe(n) enthält, mit einem solvolysierenden Mittel behandelt und/oder daß man eine erhaltene Base der Formel I durch Behandeln mit einer Säure in eines ihrer Salze umwandelt.

4. Verfahren zur Herstellung pharmazeutischer Zubereitungen, **dadurch gekennzeichnet, daß** man eine Verbindung der Formel I gemäß Anspruch 1 und/oder eines ihrer physiologisch unbedenklichen Salze zusammen mit mindestens einem festen, flüssigen oder halbflüssigen Träger- oder Hilfsstoff in eine geeignete Dosierungsform bringt.

5. Pharmazeutische Zubereitung, **gekennzeichnet durch** einen Gehalt an mindestens einer Verbindung der allgemeinen Formel I gemäß Anspruch 1 und/oder einem ihrer physiologisch unbedenklichen Salze.

6. Verwendung von Verbindungen der Formel I nach Anspruch 1 oder von deren physiologisch unbedenklichen Salzen zur Herstellung eines Arzneimittels.

7. Verwendung von Verbindungen der Formel I nach Anspruch 1 zur Herstellung von Arzneimitteln zur Behandlung von Arrhythmien, Angina pectoris, Infarkten sowie zur präventiven Behandlung der genannten Indikationen.

8. Verwendung von Verbindungen der Formel I nach Anspruch 1 zur Herstellung von Arzneimitteln zur Behandlung von Krankheiten, die mit dem Na⁺/H⁺-Antiport im Zusammenhang stehen und durch die Inhibierung desselben behandelt werden können.

## Claims

1. 4-Sulfonyl or 4-sulfinylbenzoylguanidine derivatives of the formula I 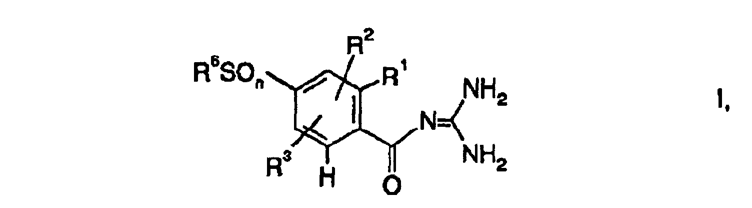 in which
R¹ is A, CF₃, CH₂F, CHF₂, C₂F₅, CN, NO₂ or Hal,
R² is -SOₙ-R⁶, -SO₂NR⁴R⁵, NO₂ or CF₃,
R³ is H, Hal, A, OH, OA, CN, NO₂, CF₃, CH₂F, CHF₂, C₂F₅ or CH₂CF₃,
R⁴ and R⁵ are each, independently of one another, H, A, cycloalkyl having 5 to 7 C atoms, cycloalkylmethyl having 4 to 8 C atoms, Ph or -CH₂-Ph,
or else
R⁴ and R⁵ together are also alkylene having 4 to 5 C atoms, where a CH₂ group may also be replaced by O, S, NH, N-A or N-CH₂-Ph,
R⁵ is A, Ph, Het or C₃-C₇-cycloalkyl;
Het is a mono- or bicyclic saturated, unsaturated or aromatic heterocycle having from 1 to 4 N, O and/or S atoms, bonded via N or C, which may be unsubstituted or mono-, di- or trisubstituted by Hal, SH, SA, NH₂, NHA, NA₂, CF₃, A, OH, OA, CN, NO₂, NHA, NA₂ and/or carbonyl oxygen,
A is alkyl having from 1 to 6 C atoms,
Hal is F, Cl, Br or l,
Ph is phenyl which is unsubstituted or mono-, di- or trisubstituted by A, OA, NR⁴R⁵, F, Cl, Br, I or CF₃, and
n is 1 or 2,
and physiologically acceptable salts thereof.

2. (a) N-Diaminomethylene-2-methyl-4,5-di(methylsulfonyl)benzamide;
(b) N-diaminomethylene-2-methyl-4-phenylsulfonyl-5-methylsulfonylbenzamide;
(c) N-diaminomethylene-2-methyl-4-tert-butylsulfonyl-5-methylsulfonylbenzamide;
(d) N-diaminomethylene-2-ethyl-4,5-di(methylsulfonyl)benzamide;
(e) N-diaminomethylene-2-ethyl-4-cyclopentylsulfonylbenzamide;
(f) N-diaminomethylene-2-methyl-4-cyclohexylsulfonyl-5-methylsulfonylbenzamide;
(g) N-diaminomethylene-2-methyl-4-methylsulfonyl-5-p-chlorophenylsulfonylbenzamide
according to Claim 1, and physiologically acceptable salts thereof.

3. Process for the preparation of alkylbenzoylguanidine derivatives of the formula I according to Claim 1 and salts thereof, **characterised in that** a compound of the formula II 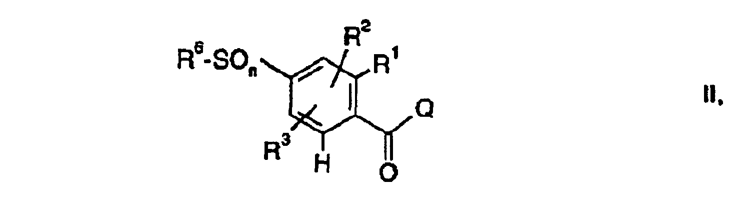 in which R¹, R², R³ and R⁶ as well as n are as defined above
and
Q is Cl, Br, OA, O-CO-A, O-CO-Ph, OH or another reactive esterified OH group or readily nucleophilically substitutable leaving group,
is reacted with guanidine,
or **in that** a benzoylguanidine of the formula III 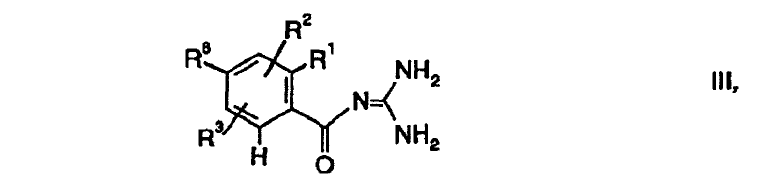 in which R¹, R² and R³ are as defined above,
and
R⁸ is F, Cl, Br, I or H,
is reacted with a hydrocarbon compound of the formula IV
R⁶SOₙ-X (IV)
in which R⁶ is as defined above, and
X is H, Cl, Br or I,
in the presence of a catalyst, after prior metallation or transmetallation,
or **in that** a benzoylguanidine of the formula V 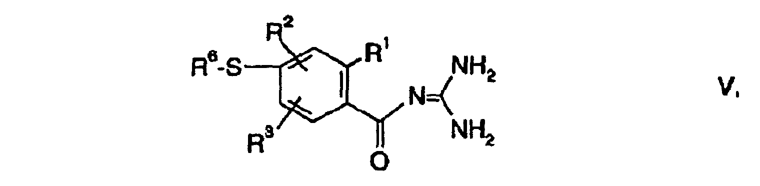 is reacted with a suitable oxidant,
or **in that** a compound which otherwise conforms to the formula I, but which contains one or more reducible group(s) and/or one or more additional C-C and/or C-N bond(s) instead of one or more hydrogen atoms is treated with a reducing agent,
or **in that** a compound which otherwise conforms to the formula I, but which contains one or more solvolysable group(s) instead of one or more hydrogen atoms is treated with a solvolysing agent,
and/or **in that** a resultant base of the formula I is converted into one of its salts by treatment with an acid.

4. Process for the preparation of pharmaceutical preparations, **characterised in that** a compound of the formula I according to Claim 1 and/or one of its physiologically acceptable salts is converted into a suitable dosage form together with at least one solid, liquid or semiliquid excipient or adjuvant.

5. Pharmaceutical preparation, **characterised by** a content of at least one compound of the general formula I according to Claim 1 and/or one of its physiologically acceptable salts.

6. Use of compounds of the formula I according to Claim 1 or physiologically acceptable salts thereof for the preparation of a medicament.

7. Use of compounds of the formula I according to Claim 1 for the preparation of medicaments for the treatment of arrhythmias, angina pectoris, infarctions and for the preventative treatment of the said indications.

8. Use of compounds of the formula I according to Claim 1 for the preparation of medicaments for the treatment of diseases which are associated with the Na⁺/H⁺ antiport and can be treated by inhibition thereof.

## Revendications

1. Dérivés de 4-sulfonyle ou de 4-sulfinylbenzoylguanidine de formule 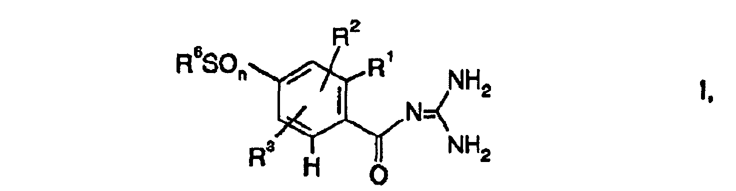 dans laquelle
R¹ désigne A, CF₃, CH₂F, CHF₂, C₂F₅, CN, NO₂ ou Hal,
R² désigne -SOₙ-R⁶, -SO₂NR⁴R⁵, NO₂ ou CF₃,
R³ désigne H, Hal, A, OH, OA, CN, NO₂, CF₃, CH₂F, CHF₂, C₂F₅ ou CH₂CF₃,
R⁴ et R⁵ chacun, indépendamment l'un de l'autre, désignent H, A, cycloalkyle ayant de 5 à 7 atomes de C, cycloalkylméthyle ayant de 4 à 8 atomes de C, Ph ou -CH₂-Ph,
ou bien
R⁴ et R⁵ ensemble désignent également alkylène ayant de 4 à 5 atomes de C, où un groupement CH₂ peut également être remplacé par O, S, NH, N-A ou N-CH₂-Ph,
R⁵ désigne A, Ph, Hét ou cycloalkyle en C₃-C₇;
Hét désigne un héterocycle mono- ou bicyclique saturé, insaturé ou aromatique ayant de 1 à 4 atomes de N, de O et/ou de S, lié par l'intermédiaire de N ou de C, pouvant être non substitué ou mono-, di- ou trisubstitué par Hal, SH, SA, NH₂, NHA, NA₂, CF₃, A, OH, OA, CN, NO₂, NHA, NA₂ et/ou oxygène carbonyle,
A désigne alkyle ayant de 1 à 6 atomes de C,
Hal désigne F, Cl, Br ou l,
Ph désigne phényle qui est non substitué ou mono-, di- ou trisubstitué par A, OA, NR⁴R⁵, F, Cl, Br, l ou CF₃, et
n désigne 1 ou 2,
et les sels physiologiquement acceptables de ceux-ci.

2. (a) N-diaminométhylène-2-méthyl-4,5-di(méthylsulfonyl)benzamide;
(b) N-diaminométhylène-2-méthyl-4-phénylsulfonyl-5-méthylsulfonylbenzamide;
(c) N-diaminométhylène-2-méthyl-4-tertio-butylsulfonyl-5-méthylsulfonylbenzamide ;
(d) N-diaminométhylène-2-éthyl-4,5-di(méthylsulfonyl)benzamide ;
(e) N-diaminométhylène-2-éthyl-4-cyclopentylsulfonylbenzamide ;
(f) N-diaminométhyléne-2-méthyl-4-cyclohexylsulfonyl-5-méthylsulfonylbenzamide ;
(g) N-diaminométhylène-2-méthyl-4-méthylsulfonyl-5-p-chlorophénylsulfonylbenzamide
selon la revendication 1, et les sels physiologiquement acceptables de ceux-ci.

3. Procédé de préparation de dérivés d'alkylbenzoylguanidine de formule l selon la revendication 1 et de sels de ceux-ci, **caractérisé en ce qu'**un composé de formule ll 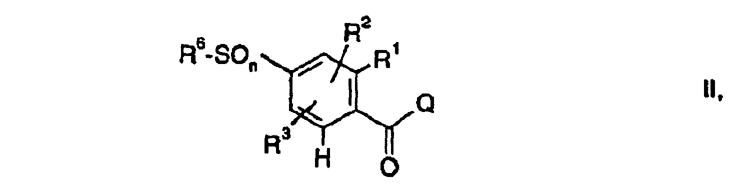 dans laquelle R¹, R², R³ et R⁶ ainsi que n sont tels que définis ci-dessus et
Q désigne Cl, Br, OA, O-CO-A, O-CO-Ph, OH ou un autre groupement OH estérifié réactif ou un groupement partant pouvant être facilement substitué de manière nucléophile,
est réagi avec de la guanidine,
ou **en ce qu'**une benzoylguanidine de formule III 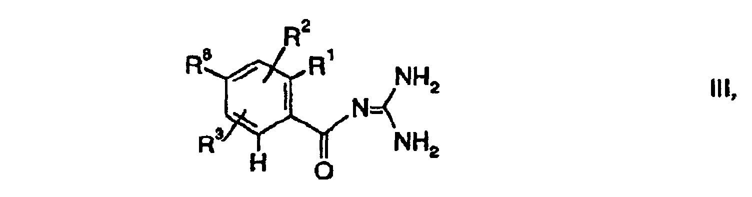 dans laquelle R¹, R² et R³ sont tels que définis ci-dessus,
et
R⁸ désigne F, Cl, Br, l ou H,
est réagie avec un composé hydrocarboné de formule IV
R⁶-SOₙ-X (IV)
dans laquelle R⁶ est tel que défini ci-dessus, et
X est H, Cl, Br ou I,
en présence d'un catalyseur, après une métallation ou transmétallation préalable,
ou **en ce qu'**une benzoylguanidine de formule V 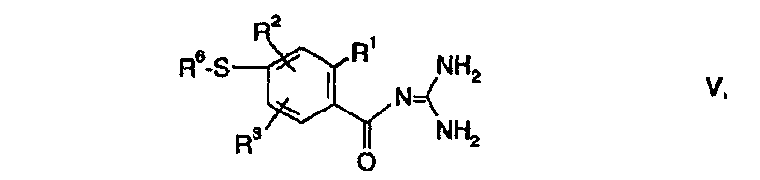 est réagie avec un oxydant convenable,
ou **en ce qu'**un composé se conformant par ailleurs à la formule l, mais qui contient un ou plusieurs groupements pouvant être réduits et/ou une ou plusieurs liaisons C-C et/ou C-N supplémentaires au lieu d'un ou plusieurs atomes d'hydrogène est traité par un agent réducteur,
ou **en ce qu'**un composé se conformant par ailleurs à la formule l, mais qui contient un ou plusieurs groupements solvolysables au lieu d'un ou plusieurs atomes d'hydrogène est traité par un agent solvolysant,
et/ou **en ce qu'**une base résultante de formule l est convertie en l'un de ses sels par un traitement par un acide.

4. Procédé de préparation de préparations pharmaceutiques, **caractérisé en ce qu'**un composé de formule l selon la revendication 1 et/ou l'un de ses sels physiologiquement acceptables est mis sous une forme de dosage convenable conjointement avec au moins un excipient ou adjuvant solide, liquide ou semi-liquide.

5. Préparation pharmaceutique, **caractérisée par** une teneur en au moins un composé de formule générale l selon la revendication 1 et/ou en l'un de ses sels physiologiquement acceptables.

6. Utilisation de composés de formule l selon la revendication 1 ou de sels physiologiquement acceptables de ceux-ci pour la préparation d'un médicament.

7. Utilisation de composés de formule l selon la revendication 1 pour la préparation de médicaments pour le traitement des arythmies, de l'angine de poitrine, des infarctus et pour le traitement préventif desdites indications.

8. Utilisation de composés de formule I selon la revendication 1 pour la préparation de médicaments pour le traitement de maladies étant liées à l'antiport Na⁺/H⁺ et pouvant être traitées par l'inhibition de celui-ci.
